# EUROPEAN PATENT SPECIFICATION

(11) **EP 0 771 785 B1**
(45) Date of publication and mention of the grant of the patent: **09.01.2002**
(21) Application number: 95307823.5
(22) Date of filing: 02.11.1995
(51) Int. Cl.: C07C 229/12, C07C 229/16, C11D 1/46, C11D 1/88, C11D 3/50

(54) **Amino ester compounds of perfume alcohols and their use in cleaning or laundry compositions**
Aminoester von Parfümalkoholen und ihre Verwendung in Reinigungs- und Waschmittelzusammensetzungen
Aminoesters d'alcools parfumants et leur utilisation dans des compositions nettoyantes et des compositions détergentes

(43) Date of publication of application: 07.05.1997
(73) Proprietor: THE PROCTER & GAMBLE COMPANY, Cincinnati, Ohio 45202 (US)
(72) Inventor: Struillou, Arnaud P., Newcastle-upon-Tyne, NE12 OGD (GB); Heinzman, Stephen W., Gosforth, Newcastle-upon-Tyne, NE3 2JP (GB); Gordon, Neil J., B-1853 Strombeek-Bever (BE)
(74) Representative: Engisch, Gautier

(56) References cited:
- EP-A- 0 310 299
- WO-A-95/04809
- WO-A-95/08976
- US-A- 3 953 605
- DATABASE WPI Section Ch, Week 9340 Derwent Publications Ltd., London, GB; Class D23, AN 93-317784 & JP-A-05 230 496 ( NIPPON SEIKA KK) , 7 September 1993
- JOURNAL OF APPLIED CHEMISTRY AND BIOTECHNOLOGY, vol. 22, no. 12, 1972 OXFORD GB, pages 1267-76, XP 000565726 D. J. ALNER ET AL. 'Esterification of Ethylenediaminetetraacetic acid acid'
- TETRAHEDRON LETT. (1977), (30), 2599-602 , ARATANI, T. ET AL 'Asymmetric synthesis of chrysanthemic acid. An application of copper carbenoid reaction'
- JOURNAL OF THE AMERICAN CHEMICAL SOCIETY, vol. 69, 1947 DC US, pages 2058-59, J. H. BILLMAN ET AL. 'Anticonvulsants. V. Esters of .gamma.-diethylamino-.alpha.-phenylbutyric acid'
- DATABASE CROSSFIRE Beilsteininformationssysteme, Frankfurt DE & J. CHEM. SOC. , vol. 99, 1911 page 2335
- DATABASE CROSSFIRE Beilsteininformationssysteme, Frankfurt DE & ZH. OBSHCH. KHIM. , vol. 26, 1956 page 1471, 1479
- CHEMICAL ABSTRACTS, vol. 85, no. 24, 13 December 1976 Columbus, Ohio, US; abstract no. 179402, KASANO, MASANOBU ET EL. page 102; & YUGAGAKU, vol. 25, no. 9, 1976 pages 557-60,

## Description

The present invention relates to amino ester compounds of perfume alcohols. More particularly, it relates to amino ester compounds of perfume alcohols in alkaline environment, such as in a hard surface cleaning composition or a laundry composition, which upon and after use will allow a slow release of the perfume component.

### Background to the invention

Consumer acceptance of cleaning and laundry products is determined not only by the performance achieved with these products but also the aesthetics associated therewith. Perfume components are therefore an important aspect of the successful formulation of such commercial products. To this end, compounds which provide a slow release of the perfume over a longer period of time than by the use of the perfume itself have been formulated. Disclosure of such compounds may be found in WO 95/04809, WO 95/08976 and pending application EP 95303762.9.

Pending application EP 95303762.9 describes betaine ester compounds of perfume alcohols which provide release of the perfume components over a long period of time. However, it has now been found that in alkaline environment such as in alkaline product, the described components hydrolyse upon storage to release their perfume component, therefore reducing the amount of perfume alcohol released upon and after the washing or cleaning process. By alkaline environment, it is meant a pH value greater or equal to 7.0.

The formulator of a laundry and/or cleaning compositions is thus faced with the challenge of formulating a compound which is stable in an alkaline environment but which still produces a slow release of the perfume alcohol upon and after the washing or cleaning process.

The Applicant has now found that the provision of amino ester compounds of perfume alcohols overcomes the problem. Such amino ester compounds of perfume alcohols have been found to be stable in alkaline environment.

Accordingly, the slow release concept may be applied to other active alcohol components such as a flavour alcohol ingredient, a pharmaceutical alcohol active or a biocontrol alcohol agent and any other active alcohol component where a slow release of said active component is necessary.

Therefore, the present invention encompasses amino ester compounds of active alcohol components.

Not to be bound by theory, it is believed that the amino ester compounds of the invention are stable in alkaline environment, and undergo protonation of at least one of their nitrogen atoms upon a consecutive pH drop of the environment and/or upon a consecutive high dilution of said environment to form the respective betaine ester compound which will hydrolyse and release the active alcohol (e.g. perfume). The pH drop may occur by any suitable means, preferably by contact with water upon rinsing processes or dilution steps.

For the purpose of the invention, the term "alkaline composition" includes granular compositions as well as liquid compositions such as aqueous compositions, said composition having a pH value greater or equal to 7.0, whereby the pH is measured at 20°C in the neat liquid product or in a 1% solution with distilled water when the composition is a solid.

By pH drop is meant a decrease of the pH measured in the environment of at least 0.5 unit versus the original pH of the composition.

By high dilution is meant a dilution of said alkaline environment by at least a factor 10, that is 1 in 10 where 10 is the final volume, that is Volume of alkaline solution and Volume of dilution.

By "slow release" is meant release of the active component (e.g. perfume) over a longer period of time than by the use of the active (e.g. perfume) itself.

According to the present invention, there is provided novel amino ester compounds of active alcohols used as precursors of the corresponding betaine ester compounds.

According to the present invention, there is provided amino ester compounds of active alcohols which are stable in alkaline compositions and provide an efficient slow release of the active component upon and after washing or cleaning processes.

According to the present invention, there is provided a method for producing a release of the active component from said composition and which comprises contacting said alkaline composition comprising the amino ester compound of active alcohols with a material so that within the resulting composition is obtained the protonation of at least one of the nitrogen atom of said amino ester compound which thereafter results in the slow release of the active component.

### Summary of the invention

The present invention relates to amino ester compounds of active alcohols having the formula: 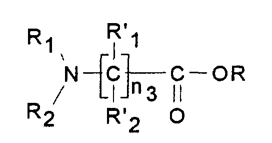 wherein each R'₁, R'₂, independently, is hydrogen,
wherein each R₁, R₂, independently, is selected from hydrogen, alkyl group, aryl group, 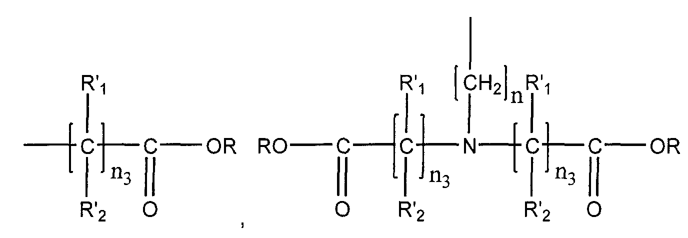 and 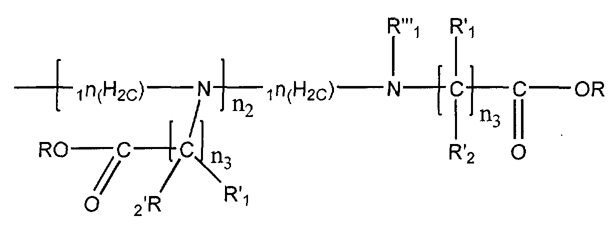
wherein each R'''₁ is selected from hydrogen, alkyl group, aryl group and
wherein each n, n₁, independently is an integer lying in the range from 1 to 20, and
wherein each n₂ is an integer lying in the range of 1 to 6,
wherein each n₃, independently, is an integer lying in the range from 1 to 3, and
wherein each R, independently, is an organic chain of an active alcohol component, said active alcohol being selected from flavour alcohols and perfume alcohols; with the proviso that at least one of R₁ and/or R₂ is: 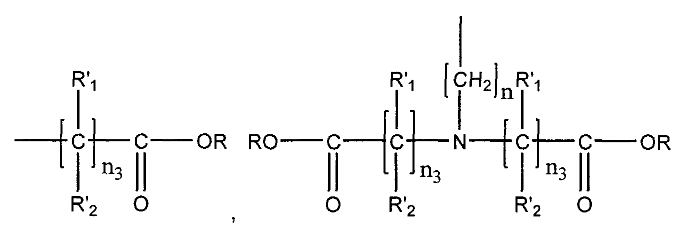
or 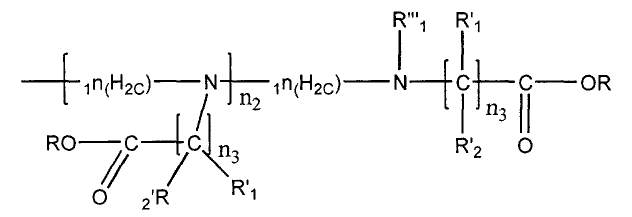 and that the compound is not:

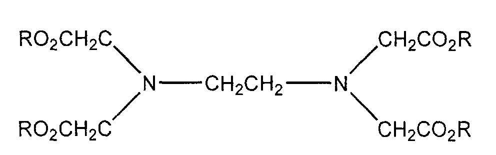

For clarity purpose, in each formula the groups R'₁ and R'₂ are included within the brackets of [C]ₙ₃ so that this should be read [CR'₁ R'₂]ₙ₃.

In another aspect of the invention, there is provided a process for preparing such compounds.

In another aspect of the invention, there is provided the use of said amino ester compounds as precursors of the corresponding betaine esters.

In a further aspect of the invention, there is provided a composition comprising a amino ester compound as defined above, said composition being alkaline(pH>7.0). A process for preparing such a composition is also provided.

In a further aspect of the invention, there is provided a method for slowly releasing an active alcohol component, preferably a perfume alcohol component from said composition which comprises contacting said composition comprising a amino ester compound as defined above with a material so that at least one of the nitrogen atom of said amino ester compound is protonated.

### Detailed description of the invention

### Amino ester compounds of active alcohols

The essential component of the invention is a amino ester compound of active alcohols.
Amino ester compounds of active alcohols of the invention have the general formula: 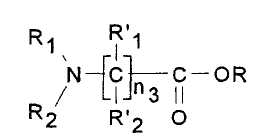 wherein each R'₁, R'₂ is hydrogen,
wherein each R₁, R₂, independently, is selected from hydrogen, alkyl group, aryl group, 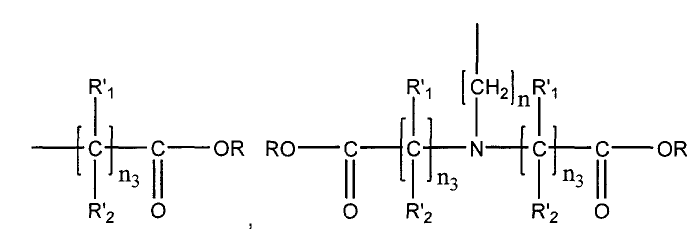 and 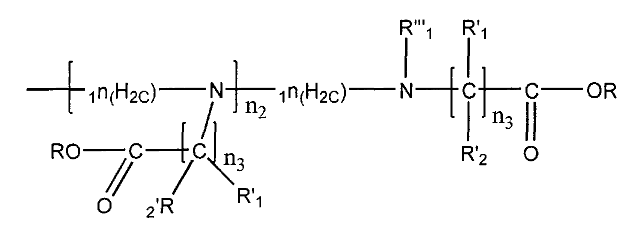
wherein each R'''₁ is selected from hydrogen, alkyl group, aryl group and
wherein each n, n₁, independently is an integer lying in the range from 1 to 20, and
wherein each n₂ is an integer lying in the range of 1 to 6,
wherein each n₃, independently, is an integer lying in the range from 1 to 3, and
wherein each R, independently, is an organic chain of an active alcohol component, said active alcohol being selected from flavour alcohols and perfume alcohols; with the proviso that at least one of R₁ and/or R₂ is: 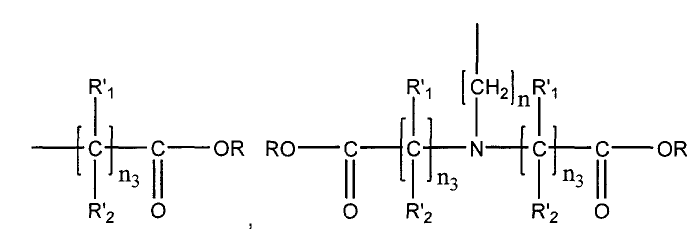
or 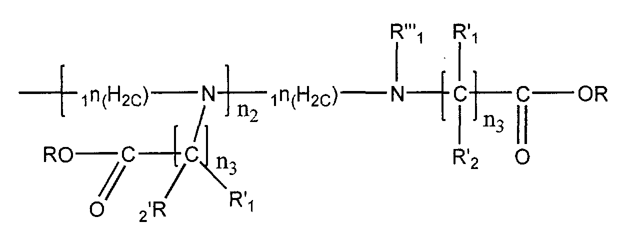 and that the compound is not:

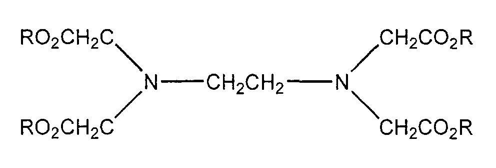

Preferred compounds of formula above are those wherein R₁ is an alkyl group, R₂ is an hydrogen and wherein R is as defined above.

Other preferred compounds of formula above are those wherein each R₁, R₂ independently is an alkyl group and wherein R is as defined above.

Other preferred compounds of formula above are those wherein R₁ is an alkyl group, R₂ is a methyl and wherein R is as defined above.

Other preferred compounds of formula above are those wherein R₁ is an hydrogen, R₂ is 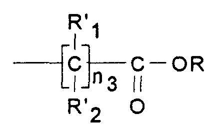 and wherein n₃ and R are as defined above.

Other preferred compounds of formula above are those wherein each R₁, R₂ is 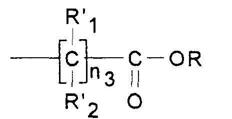 and wherein n₃ and R are as defined above.

Other preferred compounds of formula above are those wherein R₁ is 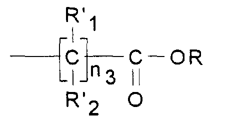 and R₂ is 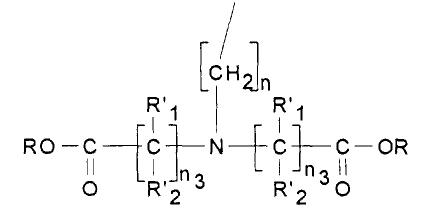 wherein each n, n₃ and R, independently, are as defined above.

Other preferred compounds of formula above are those wherein each R₁, R₂ is 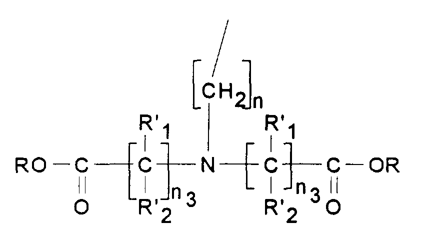 wherein each n, n₃ and R, independently, are as defined above.

Other preferred compounds of formula above are those wherein R₁ is 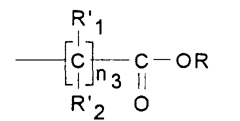 and R₂ is 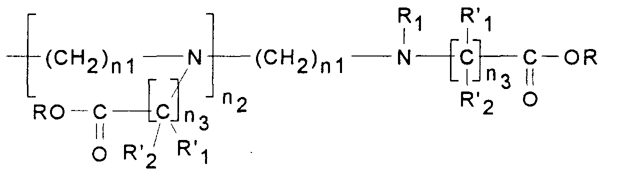 wherein each n₁, n₂, n₃ and R, independently, are as defined above.

Still other preferred compounds of formula above are those wherein R₁ and R₂ are 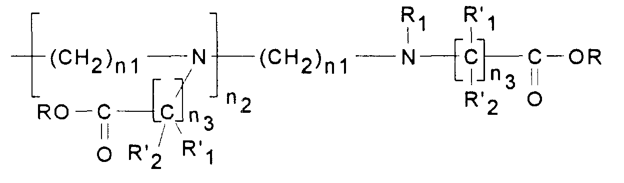 wherein each n₁, n₂, n₃ and R, independently, are as defined above.

Preferably each n, n1, independently, is an integer lying in the range 1 to 10 and more preferably has the value 2 or 3.

For the above mentioned compounds, the R group is the organic chain of an active alcohol, said active alcohol being selected from a flavour alcohol ingredient, a pharmaceutical alcohol active, a biocontrol alcohol agent, a perfume alcohol component and mixtures thereof.

Flavour ingredients include spices, flavour enhancers that contribute to the overall flavour perception.

Pharmaceutical actives include drugs.

Biocontrol agents include biocides, antimicrobials, bactericides, fungicides, algaecides, mildewcides, disinfectants, antiseptics, insecticides, vermicides, plant growth hormones.

Perfume alcohol components include components having odoriferous properties.

Preferably, for the above mentioned compounds, the R group is the organic chain of a perfume alcohol, said alcohol being selected from 2-phenoxyethanol, phenylethylalcohol, geraniol, citronellol, 3-methyl-5-phenyl-1-pentanol, 2,4-dimethyl-3-cyclohexene-1-methanol, linalool, tetrahydrolinalool, 1,2-dihydromyrcenol, hydroxycitronellal, farnesol, menthol, eugenol, vanilin, cis-3-hexenol, terpineol and mixtures thereof.
Preferred R groups for the purpose of the invention are the organic chain of geraniol and/or citronellol.

Preferred compounds from the one mentioned above include:
geranyl N-dodecylaminoacetate, N,N-di(geranyl acetate)-N-dodecylamine, N,N,N-tri(geranyl acetate)amine, 1,3-diaminopropane-N,N,N',N'-tetra(geranyl acetate), citronellyl N-Dodecylaminoacetate, N,N-di(citronellyl acetate)-N-dodecylamine, 1,3-diaminopropane-N,N,N',N'-tetra(citronellyl acetate) and mixtures thereof.

In a preferred embodiment of the invention, the beta-amino ester compounds of active alcohols, preferably perfume alcohols are used as precursors of betaine ester compounds.

### Process for preparing beta-amino ester compounds of active alcohols

The present invention also relates to a process for preparing beta-amino ester compounds of active alcohols, preferably perfume alcohols.

Such process comprises the step of:
a) esterifying the alcohol into its halogenoacetate ester form in presence of an amine, and
b) reacting said halogenoacetate ester with the desired amine in presence of a sterically hindered amine.

If in step a), the chloroacetate ester is wanted, then either chloroacetyl chloride or chloroacetic anhydride can be used to esterify the alcohol. And if the bromoacetate ester is wanted, bromoacetyl bromide can be used to esterify the alcohol. This esterification step is done using an amine (preferably a tertiary amine such as triethylamine or tributylamine, a pyridine derivative such as pyridine or 4-dimethylaminopyridine or a sterically hindered secondary amine such as diisopropylamine or diisobutylamine). The amine is used in the range 0.05 to 5 equivalent, preferably 0.8 to 1.5 equivalent. Preferred solvents for the reaction are non-polar aprotic solvent such as hexane, heptane, petroleum ether, ether, toluene, xylene, chloroform or dichloromethane.

The second step b) of the synthesis is to react the halogenoacetate ester with the desired amine, in presence of an acid acceptor, preferably a sterically hindered amine such as ammonia, butylamine, dodecylamine, aniline, tallow amine, dimethyl amine, diethyl amine, dipropyl amine, dibutyl amine, morpholine, diisopropylamine, diisobutylamine, disec-butylamine, triethylamine, tributylamine, and derivatives thereof, used as acid-acceptor. A preferred sterically hindered amine is diisopropylamine or methyldiisopropylamine. The amine is used in the range 0.05 to 5 equivalent, preferably 0.8 to 1.5 equivalent, compared to the halogenoacetate ester. Inorganic acid acceptors such as sodium hydrogen carbonate or sodium carbonate can also be used instead of the sterically hindered amine.

Preferred solvents of step b) for the preparation of N,N-di(geranyl acetate)-N-dodecylamine, 1,3-diaminopropane-N,N,N',N'-tetra(geranyl acetate), N,N-di(citronellyl acetate)-N-dodecylamine, 1,3-diaminopropane-N,N,N',N'-tetra(citronellyl acetate) are polar aprotic solvents such as acetone, dimethylformamide, dimethylsulfoxide, acetonitrile, sulfolane, N-methylpyrrolidone.

### Alkaline compositions

The present amino ester compounds of active alcohols are preferably incorporated in an alkaline composition of pH value greater than 7.0, measured at 20°C in the neat composition where the composition is an alkaline aqueous composition, or measured as a 1 % solution in distilled water at 20°C where the alkaline composition is a solid composition.

Where the active alcohol is a perfume alcohol, suitable alkaline compositions of the invention include laundry and cleaning compositions, which are typically used for laundering fabrics and cleaning hard surfaces such as floors, dishware and other surfaces in need of cleaning and/or disinfecting.

Preferably, levels of incorporation of said amino ester compounds of perfume alcohols, into the alkaline composition are of from 0.01% to 10% by weight of the total composition, more preferably 0.05% to 5%, and most preferably from 0.1% to 2%, by weight of the total composition.

Incorporation of the amino ester compound into the alkaline composition can be carried out in a number of ways such as:
1)-premixing said amino ester compound with any other additional perfume ingredients and thereafter mixing said premix with stirring to the composition, or
2)-addition with stirring of said amino ester compound per se to the composition, or
any other means known in the art such as adsorption onto a carrier before incorporation, spray-on incorporation.

When incorporated in such alkaline compositions, the composition can comprise optional components such as one or more surfactant components.

### Surfactants

The total amount of surfactants will be generally up to 70%, typically 1 to 55%, preferably 1 to 30%, more preferably 3 to 25% and especially 5 to 20% by weight of the total composition.

A typical listing of anionic, nonionic, ampholytic, and zwitterionic classes, and species of these surfactants, is given in U.S.P. 3,929,678 issued to Laughlin and Heuring on December 30, 1975. Further examples are given in "Surface Active Agents and Detergents" (Vol. I and II by Schwartz, Perry and Berch). A list of suitable cationic surfactants is given in U.S.P. 4,259,217 issued to Murphy on March 31, 1981.

Where present, ampholytic, amphoteric and zwitterionic surfactants are generally used in combination with one or more anionic and/or nonionic surfactants.

### Anionic surfactant

Essentially any anionic surfactants useful for detersive purposes can be included in the compositions. These can include salts (including, for example, sodium, potassium, ammonium, and substituted ammonium salts such as mono-, di- and triethanolamine salts) of the anionic sulphate, sulfonate, carboxylate and sarcosinate surfactants.

Other anionic surfactants include the isethionates such as the acyl isethionates, N-acyl taurates, fatty acid amides of methyl tauride, alkyl succinates and sulfosuccinates, monoesters of sulfosuccinate (especially saturated and unsaturated C₁₂-C₁₈ monoesters) diesters of sulfosuccinate (especially saturated and unsaturated C₆-C₁₄ diesters), N-acyl sarcosinates. Resin acids and hydrogenated resin acids are also suitable, such as rosin, hydrogenated rosin, and resin acids and hydrogenated resin acids present in or derived from tallow oil.

### Anionic sulphate surfactant

Anionic sulphate surfactants suitable for use herein include the linear and branched primary alkyl sulphates, alkyl ethoxysulphates, fatty oleoyl glycerol sulfates, alkyl phenol ethylene oxide ether sulfates, the C₅-C₁₇ acyl-N-(C₁-C₄ alkyl) and -N-(C₁-C₂ hydroxyalkyl) glucamine sulphates, and sulphates of alkylpolysaccharides such as the sulphates of alkylpolyglucoside (the nonionic nonsulphated compounds being described herein).

Alkyl ethoxysulphate surfactants are preferably selected from the group consisting of the C₆-C₁₈ alkyl sulphates which have been ethoxylated with from about 0.5 to about 20 moles of ethylene oxide per molecule. More preferably, the alkyl ethoxysulphate surfactant is a C₆-C₁₈ alkyl sulphate which has been ethoxylated with from about 0.5 to about 20, preferably from about 0.5 to about 5, moles of ethylene oxide per molecule.

### Anionic sulfonate surfactant

Anionic sulfonate surfactants suitable for use herein include the salts of C₅-C₂₀ linear alkylbenzene sulfonates, alkyl ester sulfonates, C₆-C₂₂ primary or secondary alkane sulfonates, C₆-C₂₄ olefin sulfonates, sulfonated polycarboxylic acids, alkyl glycerol sulfonates, fatty acyl glycerol sulfonates, fatty oleyl glycerol sulfonates, and any mixtures thereof.

### Anionic carboxylate surfactant

Anionic carboxylate surfactants suitable for use herein include the alkyl ethoxy carboxylates, the alkyl polyethoxy polycarboxylate surfactants and the soaps ('alkyl carboxyls'), especially certain secondary soaps as described herein.

Preferred alkyl ethoxy carboxylates for use herein include those with the formula RO(CH₂CH₂0)ₓ CH₂C00⁻M⁺ wherein R is a C₆ to C₁₈ alkyl group, x ranges from O to 10, and the ethoxylate distribution is such that, on a weight basis, the amount of material where x is 0 is less than about 20 %, and the amount of material where x is greater than 7, is less than about 25 %, the average x is from about 2 to 4 when the average R is C₁₃ or less, and the average x is from about 3 to 10 when the average R is greater than C₁₃, and M is a cation, preferably chosen from alkali metal, alkaline earth metal, ammonium, mono-, di-, and tri-ethanol-ammonium, most preferably from sodium, potassium, ammonium and mixtures thereof with magnesium ions. The preferred alkyl ethoxy carboxylates are those where R is a C₁₂ to C₁₈ alkyl group.

Alkyl polyethoxy polycarboxylate surfactants suitable for use herein include those having the formula RO-(CHR₁-CHR₂-O)-R₃ wherein R is a C₆ to C₁₈ alkyl group, x is from 1 to 25, R₁ and R₂ are selected from the group consisting of hydrogen, methyl acid radical, succinic acid radical, hydroxysuccinic acid radical, and mixtures thereof, wherein at least one R₁ or R₂ is a succinic acid radical or hydroxysuccinic acid radical, and R₃ is selected from the group consisting of hydrogen, substituted or unsubstituted hydrocarbon having between 1 and 8 carbon atoms, and mixtures thereof.

### Anionic secondary soap surfactant

Preferred soap surfactants are secondary soap surfactants which contain a carboxyl unit connected to a secondary carbon. The secondary carbon can be in a ring structure, e.g. as in p-octyl benzoic acid, or as in alkyl-substituted cyclohexyl carboxylates. The secondary soap surfactants should preferably contain no ether linkages, no ester linkages and no hydroxyl groups. There should preferably be no nitrogen atoms in the head-group (amphiphilic portion). The secondary soap surfactants usually contain 11-15 total carbon atoms, although slightly more (e.g., up to 16) can be tolerated, e.g. p-octyl benzoic acid.

The following general structures further illustrate some of the preferred secondary soap surfactants:
- A.: A highly preferred class of secondary soaps comprises the secondary carboxyl materials of the formula R³ CH(R⁴)COOM, wherein R³ is CH₃(CH₂)x and R⁴ is CH₃(CH₂)y, wherein y can be O or an integer from 1 to 4, x is an integer from 4 to 10 and the sum of (x + y) is 6-10, preferably 7-9, most preferably 8.
- B.: Another preferred class of secondary soaps comprises those carboxyl compounds wherein the carboxyl substituent is on a ring hydrocarbyl unit, i.e., secondary soaps of the formula R⁵-R⁶-COOM, wherein R⁵ is C⁷-C¹⁰, preferably C⁸-C⁹, alkyl or alkenyl and R⁶ is a ring structure, such as benzene, cyclopentane and cyclohexane. (Note: R⁵ can be in the ortho, meta or para position relative to the carboxyl on the ring.)
- C.: Still another preferred class of secondary soaps comprises secondary carboxyl compounds of the formula CH₃(CHR)ₖ-(CH₂)ₘ-(CHR)ₙ-CH(COOM)(CHR)ₒ-(CH2)ₚ-(CHR)_{q}-CH₃, wherein each R is C₁-C₄ alkyl, wherein k, n, o, q are integers in the range of 0-8, provided that the total number of carbon atoms (including the carboxylate) is in the range of 10 to 18.

In each of the above formulas A, B and C, the species M can be any suitable, especially water-solubilizing, counterion.

Especially preferred secondary soap surfactants for use herein are water-soluble members selected from the group consisting of the water-soluble salts of 2-methyl-1-undecanoic acid, 2-ethyl-1-decanoic acid, 2-propyl-1-nonanoic acid, 2-butyl-1-octanoic acid and 2-pentyl-1-heptanoic acid.

### Alkali metal sarcosinate surfactant

Other suitable anionic surfactants are the alkali metal sarcosinates of formula R-CON (R¹) CH₂ COOM, wherein R is a C₅-C₁₇ linear or branched alkyl or alkenyl group, R¹ is a C₁-C₄ alkyl group and M is an alkali metal ion. Preferred examples are the myristyl and oleoyl methyl sarcosinates in the form of their sodium salts.

Not to be bound by theory, it is believed that where present anionic surfactants enhance the stability of the beta-amino ester compound. The anionic surfactants in alkaline compositions form micelles which incorporate the beta-amino ester compound thus, protecting the hydrolysis of the ester bond.

### Nonionic surfactant

Essentially any nonionic surfactants useful for detersive purposes, especially for hard surfaces cleaning, can be included in the compositions. Exemplary, non-limiting classes of useful nonionic surfactants are listed below.

### Nonionic polyhydroxy fatty acid amide surfactant

Polyhydroxy fatty acid amides suitable for use herein are those having the structural formula R²CONR¹Z wherein : R1 is H, C₁-C₄ hydrocarbyl, 2-hydroxy ethyl, 2-hydroxy propyl, or a mixture thereof, preferable C1-C4 alkyl, more preferably C₁ or C₂ alkyl, most preferably C₁ alkyl (i.e., methyl); and R₂ is a C₅-C₃₁ hydrocarbyl, preferably straight-chain C₅-C₁₉ alkyl or alkenyl, more preferably straight-chain C₉-C₁₇ alkyl or alkenyl, most preferably straight-chain C₁₁-C₁₇ alkyl or alkenyl, or mixture thereof; and Z is a polyhydroxyhydrocarbyl having a linear hydrocarbyl chain with at least 3 hydroxyls directly connected to the chain, or an alkoxylated derivative (preferably ethoxylated or propoxylated) thereof. Z preferably will be derived from a reducing sugar in a reductive amination reaction; more preferably Z is a glycityl.

### Nonionic condensates of alkyl phenols

The polyethylene, polypropylene, and polybutylene oxide condensates of alkyl phenols are suitable for use herein. In general, the polyethylene oxide condensates are preferred. These compounds include the condensation products of alkyl phenols having an alkyl group containing from about 6 to about 18 carbon atoms in either a straight chain or branched chain configuration with the alkylene oxide.

### Nonionic ethoxylated alcohol surfactant

The alkyl ethoxylate condensation products of aliphatic alcohols with from about 1 to about 25 moles of ethylene oxide are suitable for use herein. The alkyl chain of the aliphatic alcohol can either be straight or branched, primary or secondary, and generally contains from 6 to 22 carbon atoms. Particularly preferred are the condensation products of alcohols having an alkyl group containing from 8 to 20 carbon atoms with from about 2 to about 10 moles of ethylene oxide per mole of alcohol.

### Nonionic ethoxylated /propoxylated fatty alcohol surfactant

The ethoxylated C₆-C₁₈ fatty alcohols and C₆-C₁₈ mixed ethoxylated/propoxylated fatty alcohols are suitable surfactants for use herein, particularly where water soluble. Preferably the ethoxylated fatty alcohols are the C₁₀-C₁₈ ethoxylated fatty alcohols with a degree of ethoxylation of from 3 to 50, most preferably these are the C₁₂-C₁₈ ethoxylated fatty alcohols with a degree of ethoxylation from 3 to 40. Preferably the mixed ethoxylated/propoxylated fatty alcohols have an alkyl chain length of from 10 to 18 carbon atoms, a degree of ethoxylation of from 3 to 30 and a degree of propoxylation of from 1 to 10.

### Nonionic EO/PO condensates with propylene glycol

The condensation products of ethylene oxide with a hydrophobic base formed by the condensation of propylene oxide with propylene glycol are suitable for use herein. The hydrophobic portion of these compounds preferably has a molecular weight of from about 1500 to about 1800 and exhibits water insolubility. Examples of compounds of this type include certain of the commercially-available Pluronic™ surfactants, marketed by BASF.

### Nonionic EO condensation products with propylene oxide/ethylene diamine adducts

The condensation products of ethylene oxide with the product resulting from the reaction of propylene oxide and ethylenediamine are suitable for use herein. The hydrophobic moiety of these products consists of the reaction product of ethylenediamine and excess propylene oxide, and generally has a molecular weight of from about 2500 to about 3000. Examples of this type of nonionic surfactant include certain of the commercially available Tetronic™ compounds, marketed by BASF.

### Nonionic alkylpolysaccharide surfactant

Suitable alkylpolysaccharides for use herein are disclosed in U.S. Patent 4,565,647, Llenado, issued January 21, 1986, having a hydrophobic group containing from about 6 to about 30 carbon atoms, preferably from about 10 to about 16 carbon atoms and a polysaccharide, e.g., a polyglycoside, hydrophilic group containing from about 1.3 to about 10, preferably from about 1.3 to about 3, most preferably from about 1.3 to about 2.7 saccharide units. Any reducing saccharide containing 5 or 6 carbon atoms can be used, e.g., glucose, galactose and galactosyl moieties can be substituted for the glucosyl moieties. (Optionally the hydrophobic group is attached at the 2-, 3-, 4-, etc. positions thus giving a glucose or galactose as opposed to a glucoside or galactoside.) The intersaccharide bonds can be, e.g., between the one position of the additional saccharide units and the 2-, 3-, 4-, and/or 6- positions on the preceding saccharide units.

The preferred alkylpolyglycosides have the formula

R²O(CₙH₂ₙO)t(glycosyl)ₓ

wherein R2 is selected from the group consisting of alkyl, alkylphenyl, hydroxyalkyl, hydroxyalkylphenyl, and mixtures thereof in which the alkyl groups contain from 10 to 18, preferably from 12 to 14, carbon atoms; n is 2 or 3; t is from 0 to 10, preferably 0, and X is from 1.3 to 8, preferably from 1.3 to 3, most preferably from 1.3 to 2.7. The glycosyl is preferably derived from glucose.

### Nonionic fatty acid amide surfactant

Fatty acid amide surfactants suitable for use herein are those having the formula: R⁶CON(R⁷)₂ wherein R⁶ is an alkyl group containing from 7 to 21, preferably from 9 to 17 carbon atoms and each R⁷ is selected from the group consisting of hydrogen, C₁-C₄ alkyl, C₁-C₄ hydroxyalkyl, and -(C₂H₄O)ₓH, where x is in the range of from 1 to 3.

Other conventional useful surfactants are listed in standard texts.

Optional ingredients useful for formulating such laundry and cleaning compositions according to the present invention include one or more of the following.

### Additional perfume

Additional perfume ingredients may be added to the alkaline composition. When present, the composition will comprise up to 5% by weight, more preferably from 0.1% to 1.5% by weight of additional perfume.

Additional perfumes are those odorous materials that deposit on fabrics or surfaces during the laundry or cleaning process and are detectable by people with normal olfactory sensity. Many of the perfume ingredients along with their odor corrector and their physical and chemical properties are given in "Perfume and Flavor chemicals (aroma chemicals)", Stephen Arctender, Vols. I and II, Aurthor, Montclair, H.J. and the Merck Index, 8th Edition, Merck & Co., Inc. Rahway, N.J. Perfume components and compositions can also be found in the art, e.g. US Patent Nos. 4,145,184, 4,152,272, 4,209,417 or 4,515,705.

A wide variety of chemicals are known for perfume use including materials such as aldehydes, ketones, esters and the like. More commonly, naturally occurring plant and animal oils and exudates comprising complex mixtures of various chemical components are known for use as perfume, and such materials can be used herein. Typical perfumes can comprise e.g. woody/earthy bases containing exotic materials such as sandalwood oil, civet and patchouli oil. The perfume also can be of a light floral fragrance e.g. rose or violet extract. Further the perfume can be formulated to provide desirable fruity odours e.g. lime, limon or orange.

Particular examples of optional perfume ingredients and compositions are anetole, benzaldehyde, benzyl acetate, benzyl alcohol, benzyl formate, isobornyl acetate, camphene, cis-citral (neral), citronellal, citronellol, citronellyl acetate, paracymene, decanal, dihydrolinalool, dihydromyrcenol, dimethyl phenyl carbinol, eucalyptol, geranial, geraniol, geranyl acetate, geranyl nitrile, cis-3-hexenyl acetate, hydroxycitronellal, d-limonene, linalool, linalool oxide, linalyl acetate, linalyl propionate, methyl anthranilate, alpha-methyl ionone, methyl nonyl acetaldehyde, methyl phenyl carbinyl acetate, laevo-menthyl acetate, menthone, iso-menthone, myrcene, myrcenyl acetate, myrcenol, nerol, neryl acetate, nonyl acetate, phenyl ethyl alcohol, alpha-pinene, beta-pinene, gamma-terpinene, alpha-terpineol, beta-terpineol, terpinyl acetate, vertenex (para-tertiary-butyl cyclohexyl acetate), amyl cinnamic aldehyde, iso-amyl salicylate, beta-caryophyllene, cedrene, cinnamic alcohol, couramin, dimethyl benzyl carbinyl acetate, ethyl vanillin, eugenol, iso-eugenol, flor acetate, heliotrophine, 3-cis-hexenyl salicylate, hexyl salicylate, lilial (para-tertiarybutyl-alpha-methyl hydrocinnamic aldehyde), gamma-methyl ionone, nerolidol, patchouli alcohol, phenyl hexanol, beta-selinene, trichloromethyl phenyl carbinyl acetate, triethyl citrate, vanillin, veratraldehyde, alpha-cedrene, beta-cedrene, C15H24sesquiterpenes, benzophenone, benzyl salicylate, ethylene brassylate, galaxolide (1,3,4,6,7,8-hexahydro-4,6,6,7,8,8,-hexamethyl-cyclopenta-gamma-2-benzopyran), hexyl cinnamic aldehyde, lyral (4-(4-hydroxy-4-methyl pentyl)-3-cyclohexene-10-carboxaldehyde), methyl cedrylone, methyl dihydro jasmonate, methyl-beta-naphthyl ketone, musk ambrette, musk idanone, musk ketone, musk tibetine, musk xylol, aurantiol and phenylethyl phenyl acetate and mixtures thereof.

### Builders

Detergent builders can optionally be included in the compositions herein to assist in controlling mineral hardness. Inorganic as well as organic builders can be used. Builders are typically used in fabric laundering compositions to assist in the removal of particulate soils.

The level of builder can vary widely depending upon the end use of the composition and its desired physical form. When present, the compositions will typically comprise at least about 1% builder, preferably from about 1% to about 80%. Liquid formulations typically comprise from about 5% to about 50%, more typically about 5% to about 30%, by weight, of detergent builder. Granular formulations typically comprise from about 1% to about 80%, more typically from about 5% to about 50% by weight, of the detergent builder. Lower or higher levels of builder, however, are not meant to be excluded.

Inorganic or P-containing detergent builders include, but are not limited to, the alkali metal, ammonium and alkanolammonium salts of polyphosphates (exemplified by the tripolyphosphates, pyrophosphates, and glassy polymeric meta-phosphates), phosphonates, phytic acid, silicates, carbonates (including bicarbonates and sesquicarbonates), sulphates, and aluminosilicates. However, non-phosphate builders are required in some locales. Importantly, the compositions herein function surprisingly well even in the presence of the so-called "weak" builders (as compared with phosphates) such as citrate, or in the so-called "underbuilt" situation that may occur with zeolite or layered silicate builders.

Examples of silicate builders are the alkali metal silicates, particularly those having a SiO₂:Na₂O ratio in the range 1.0:1 to 3.2:1 and layered silicates, such as the layered sodium silicates described in U.S 4,664,839. NaSKS-6 is the trademark for a crystalline layered silicate marketed by Hoechst (commonly abbreviated herein as "SKS-6"). Unlike zeolite builders, the Na SKS-6 silicate builder does not contain aluminium. NaSKS-6 has the delta-Na₂Si₂O₅ morphology form of layered silicate. It can be prepared by methods such as those described in German DE-A-3,417,649 and DE-A-3,742,043. SKS-6 is a highly preferred layered silicate for use herein, but other such layered silicates, such as those having the general formula NaMSiₓO₂ₓ₊₁·yH₂O wherein M is sodium or hydrogen, x is a number from 1.9 to 4, preferably 2, and y is a number from 0 to 20, preferably 0 can be used herein. Various other layered silicates from Hoechst include NaSKS-5, NaSKS-7 and NaSKS-11, as the alpha, beta and gamma forms. As noted above, the delta-Na₂Si₂O₅ (NaSKS-6 form) is most preferred for use herein. Other silicates may also be useful such as for example magnesium silicate, which can serve as a crispening agent in granular formulations, as a stabilizing agent for oxygen bleaches, and as a component of suds control systems.

Examples of carbonate builders are the alkaline earth and alkali metal carbonates as disclosed in German Patent Application No. 2,321,001 published on November 15, 1973.

Aluminosilicate builders are useful in the present invention. Aluminosilicate builders are of great importance in most currently marketed heavy duty granular detergent compositions, and can also be a significant builder ingredient in liquid detergent formulations. Aluminosilicate builders include those having the empirical formula:

M_{z/n}[(AlO₂)_{z}(SiO₂)_{y}]·xH₂O

wherein z and y are integers usually of at least 6, the molar ratio of z to y is in the range from 1.0 to 0, and x is an integer from 0 to about 264, and M is a Group IA or IIA element, e.g., Na, K, Mg, Ca with valence n.

Useful aluminosilicate ion exchange materials are commercially available. These aluminosilicates can be crystalline or amorphous in structure and can be naturally-occurring aluminosilicates or synthetically derived. A method for producing aluminosilicate ion exchange materials is disclosed in U.S 3,985,669. Preferred synthetic crystalline aluminosilicate ion exchange materials useful herein are available under the designations Zeolite A, Zeolite P (B), Zeolite MAP and Zeolite X. In an especially preferred embodiment, the crystalline aluminosilicate ion exchange material has the formula:

Na₁₂[(AlO₂)₁₂(SiO₂)₁₂]·xH₂O

wherein x is from about 20 to about 30, especially about 27. This material is known as Zeolite A. Dehydrated zeolites (x = 0 - 10) may also be used herein. Preferably, the aluminosilicate has a particle size of about 0.1-10 microns in diameter.

Organic detergent builders suitable for the purposes of the present invention include, but are not restricted to, a wide variety of polycarboxylate compounds. As used herein, "polycarboxylate" refers to compounds having a plurality of carboxylate groups, preferably at least 3 carboxylates. Polycarboxylate builder can generally be added to the composition in acid form, but can also be added in the form of a neutralized salt. When utilized in salt form, alkali metals, such as sodium, potassium, and lithium, or alkanolammonium salts are preferred.

Included among the polycarboxylate builders are a variety of categories of useful materials. One important category of polycarboxylate builders encompasses the ether polycarboxylates, including oxydisuccinate, as disclosed in Berg, U.S 3,128,287, U.S 3,635,830. See also "TMS/TDS" builders of U.S 4,663,071. Suitable ether polycarboxylates also include cyclic compounds, particularly alicyclic compounds, such as those described in U.S 3,923,679; 3,835,163; 4,158,635; 4,120,874 and 4,102,903.

Other useful detergency builders include the ether hydroxypolycarboxylates, copolymers of maleic anhydride with ethylene or vinyl methyl ether, 1, 3, 5-trihydroxy benzene-2, 4, 6-trisulphonic acid, and carboxymethyloxysuccinic acid, the various alkali metal, ammonium and substituted ammonium salts of polyacetic acids such as ethylenediamine tetraacetic acid and nitrilotriacetic acid, as well as polycarboxylates such as mellitic acid, pyromellitic, succinic acid, oxydisuccinic acid, polymaleic acid, benzene 1,3,5-tricarboxylic acid, carboxymethyloxysuccinic acid, and soluble salts thereof.

Citrate builders, e.g., citric acid and soluble salts thereof (particularly sodium salt), are polycarboxylate builders of particular importance for heavy duty liquid detergent formulations due to their availability from renewable resources and their biodegradability. Citrates can also be used in granular compositions, especially in combination with zeolite and/or layered silicate builders. Oxydisuccinates are also especially useful in such compositions and combinations.

Also suitable in the detergent compositions of the present invention are the 3,3-dicarboxy-4-oxa-1,6-hexanedioates and the related compounds disclosed in U.S 4,566,984. Useful succinic acid builders include the C₅-C₂₀ alkyl and alkenyl succinic acids and salts thereof. A particularly preferred compound of this type is dodecenylsuccinic acid. Specific examples of succinate builders include: laurylsuccinate, myristylsuccinate, palmitylsuccinate, 2-dodecenylsuccinate (preferred), 2-pentadecenylsuccinate, and the like. Laurylsuccinates are the preferred builders of this group, and are described in European Patent Application 86200690.5/0,200,263, published November 5, 1986.

Other suitable polycarboxylates are disclosed in U.S 4,144,226 and in U.S 3,308,067. See also U.S 3,723,322.

Fatty acids, e.g., C₁₂-C₁₈ monocarboxylic acids such as oleic acid and/or its salts, can also be incorporated into the compositions alone, or in combination with the aforesaid builders, especially citrate and/or the succinate builders, to provide additional builder activity. Such use of fatty acids will generally result in a diminution of sudsing, which should be taken into account by the formulator.

In situations where phosphorus-based builders can be used, and especially in the formulation of bars used for hand-laundering operations, the various alkali metal phosphates such as the well-known sodium tripolyphosphates, sodium pyrophosphate and sodium orthophosphate can be used. Phosphonate builders such as ethane-1-hydroxy-1,1-diphosphonate, ethylene diphosphonate, α-hydroxy-2 phenyl ethyl diphosphonate, methylene diphosphonate, vinylidene 1,1 diphosphonate , 1,2 dihydroxyethane 1,1 diphosphonate and other known phosphonates (see, for example, U.S. Patents 3,159,581; 3,213,030; 3,422,021; 3,400,148 and 3,422,137) can also be used.

### Bleaching Compounds - Bleaching Agents and Bleach Activators

The detergent compositions herein may optionally contain bleaching agents or bleaching compositions containing a bleaching agent and one or more bleach activators. When present, bleaching agents will typically be at levels of from about 1% to about 30%, more typically from about 5% to about 20%, of the detergent composition, especially for fabric laundering. If present, the amount of bleach activators will typically be from about 0.1% to about 60%, more typically from about 0.5% to about 40% of the bleaching composition comprising the bleaching agent-plus-bleach activator.

The bleaching agents used herein can be any of the bleaching agents useful for detergent compositions in textile cleaning or other cleaning purposes. These include oxygen bleaches such as hydrogen peroxide as well as other bleaching agents. Perborate bleaches, e.g., sodium perborate (e.g., mono- or tetrahydrate) can be used herein as well as percarbonate bleaches.

Another category of bleaching agent that can be used without restriction encompasses hypohalite bleaches. Hypohalite bleaches are oxidative bleaches which subsequently lead to the formation of halide ion. Hypohalites bleaches are typically at a level of from 1 % to 15% by weight, more preferably from 1 % to 10% by weight of the composition.

Common among these types of bleaches are the alkaline metal and alkaline earth metal hypochlorites, hypobromites and hypoiodites although other bleaches that are organic based sources of halide, such as chloroisocyanurates, are also applicable. Preferred bleach has the formula M(OX)y where:
M is member selected from sodium, potassium, magnesium, calcium and mixtures thereof;
O is an oxygen atom;
X is member selected from chlorine, bromine, iodine and mixtures thereof; and
y is 1 or 2 depending on the charge of M.

Preferred hypohalite bleaches for the purpose of the invention are sodium hypochlorite, potassium hypochlorite, calcium hypochlorite, magnesium hypochlorite, sodium hypobromite, potassium hypobromite, calcium hypobromite, magnesium hypobromite, sodium hypoiodite and potassium hypoiodite , more preferably sodium hypochlorite, potassium hypochlorite, calcium hypochlorite, magnesium hypochlorite, most preferably sodium hypochlorite.

Another category of bleaching agent that can be used without restriction encompasses percarboxylic acid bleaching agents and salts thereof. Suitable examples of this class of agents include magnesium monoperoxyphthalate hexahydrate, the magnesium salt of metachloro perbenzoic acid, 4-nonylamino-4-oxoperoxybutyric acid and diperoxydodecanedioic acid. Such bleaching agents are disclosed in U.S 4,483,781, U.S. Patent Application 740,446, Burns et al, filed June 3, 1985, EP 0,133,354 and U.S 4,412,934. Highly preferred bleaching agents also include 6-nonylamino-6-oxoperoxycaproic acid as described in U.S 4,634,551.

Peroxygen bleaching agents can also be used. Suitable peroxygen bleaching compounds include sodium carbonate peroxyhydrate and equivalent "percarbonate" bleaches, sodium pyrophosphate peroxyhydrate, urea peroxyhydrate, and sodium peroxide. Persulfate bleach (e.g., OXONE, manufactured commercially by DuPont) can also be used.

A preferred percarbonate bleach comprises dry particles having an average particle size in the range from about 500 micrometers to about 1,000 micrometers, not more than about 10% by weight of said particles being smaller than about 200 micrometers and not more than about 10% by weight of said particles being larger than about 1,250 micrometers. Optionally, the percarbonate can be coated with silicate, borate or water-soluble surfactants. Percarbonate is available from various commercial sources such as FMC, Solvay and Tokai Denka.

Mixtures of bleaching agents can also be used.

Peroxygen bleaching agents, the perborates, the percarbonates, etc., are preferably combined with bleach activators, which lead to the *in situ* production in aqueous solution (i.e., during the washing process) of the peroxy acid corresponding to the bleach activator. Various nonlimiting examples of activators are disclosed in U.S 4,915,854 and U.S 4,412,934. The nonanoyloxybenzene sulfonate (NOBS) and tetraacetyl ethylene diamine (TAED) activators are typical, and mixtures thereof can also be used. See also U.S 4,634,551 for other typical bleaches and activators useful herein.

Highly preferred amido-derived bleach activators are those of the formulae:

R¹N(R⁵)C(O)R²C(O)L or R¹C(O)N(R⁵)R²C(O)L

wherein R¹ is an alkyl group containing from about 6 to about 12 carbon atoms, R² is an alkylene containing from 1 to about 6 carbon atoms, R⁵ is H or alkyl, aryl, or alkaryl containing from about 1 to about 10 carbon atoms, and L is any suitable leaving group. A leaving group is any group that is displaced from the bleach activator as a consequence of the nucleophilic attack on the bleach activator by the perhydrolysis anion. A preferred leaving group is phenyl sulfonate.

Preferred examples of bleach activators of the above formulae include (6-octanamido-caproyl)oxybenzenesulfonate, (6-nonanamidocaproyl) oxybenzenesulfonate, (6-decanamido-caproyl)oxybenzenesulfonate, and mixtures thereof as described in U.S 4,634,551.

Another class of bleach activators comprises the benzoxazin-type activators disclosed in U.S 4,966,723, incorporated herein by reference. A highly preferred activator of the benzoxazin-type is:

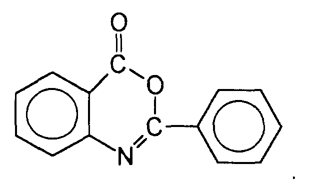

Still another class of preferred bleach activators includes the acyl lactam activators, especially acyl caprolactams and acyl valerolactams of the formulae: 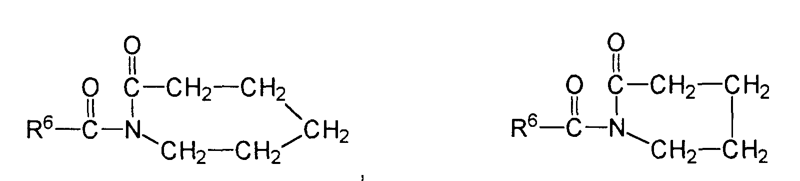 wherein R⁶ is H or an alkyl, aryl, alkoxyaryl, or alkaryl group containing from 1 to about 12 carbon atoms. Highly preferred lactam activators include benzoyl caprolactam, octanoyl caprolactam, 3,5,5-trimethylhexanoyl caprolactam, nonanoyl caprolactam, decanoyl caprolactam, undecenoyl caprolactam, benzoyl valerolactam, octanoyl valerolactam, decanoyl valerolactam, undecenoyl valerolactam, nonanoyl valerolactam, 3,5,5-trimethylhexanoyl valerolactam and mixtures thereof. See also U.S 4,545,784, which discloses acyl caprolactams, including benzoyl caprolactam, adsorbed into sodium perborate.

Bleaching agents other than oxygen bleaching agents are also known in the art and can be utilized herein. One type of non-oxygen bleaching agent of particular interest includes photoactivated bleaching agents such as the sulfonated zinc and/or aluminum phthalocyanines. See U.S 4,033,718. If used, detergent compositions will typically contain from about 0.025% to about 1.25%, by weight, of such bleaches, especially sulfonate zinc phthalocyanine.

If desired, the bleaching compounds can be catalyzed by means of a manganese compound. Such compounds are well known in the art and include, for example, the manganese-based catalysts disclosed in U.S 5,246,621, U.S 5,244,594; U.S 5,194,416; U.S 5,114,606; and EP 549,271A1, 549,272A1, 544,440A2, and 544,490A1; Preferred examples of these catalysts include Mn^{IV}₂(u-O)₃(1,4,7-trimethyl-1,4,7-triazacyclononane)₂(PF₆)₂, Mn^{III}₂(u-O)₁(u-OAc)₂(1,4,7-trimethyl-1,4,7-triazacyclononane)₂-(ClO₄)₂, Mn^{IV}₄(u-O)₆(1,4,7-triazacyclononane)₄(ClO₄)₄, Mn^{III}Mn^{IV}₄(u-O)₁(u-OAc)₂₋(1,4,7-trimethyl-1,4,7-triazacyclononane)₂(ClO₄)₃, Mn^{IV}(1,4,7-trimethyl-1,4,7-triazacyclononane)-(OCH₃)₃(PF₆), and mixtures thereof. Other metal-based bleach catalysts include those disclosed in U.S 4,430,243 and U.S 5,114,611. The use of manganese with various complex ligands to enhance bleaching is also reported in the following US Patents: 4,728,455; 5,284,944; 5,246,612; 5,256,779; 5,280,117; 5,274,147; 5,153,161; and 5,227,084.

As a practical matter, and not by way of limitation, the compositions and processes herein can be adjusted to provide on the order of at least one part per ten million of the active bleach catalyst species in the aqueous washing liquor, and will preferably provide from about 0.1 ppm to about 700 ppm, more preferably from about 1 ppm to about 500 ppm, of the catalyst species in the laundry liquor.

### Enzymes

Enzymes are included in the formulations herein for a wide variety of fabric laundering purposes, including removal of protein-based, carbohydrate-based, or triglyceride-based stains, for example, and for the prevention of refugee dye transfer, and for fabric restoration. The enzymes to be incorporated include proteases, amylases, lipases, cellulases, and peroxidases, as well as mixtures thereof. Other types of enzymes may also be included. They may be of any suitable origin, such as vegetable, animal, bacterial, fungal and yeast origin. However, their choice is governed by several factors such as pH-activity and/or stability optima, thermostability, stability versus active detergents, builders and so on. In this respect bacterial or fungal enzymes are preferred, such as bacterial amylases and proteases, and fungal cellulases.

Enzymes are normally incorporated at levels sufficient to provide up to about 5 mg by weight, more typically about 0.001 mg to about 3 mg, of active enzyme per gram of the composition. Stated otherwise, the compositions herein will typically comprise from about 0.001% to about 5%, preferably 0.01%-2% by weight of a commercial enzyme preparation. Protease enzymes are usually present in such commercial preparations at levels sufficient to provide from 0.005 to 0.1 Anson units (AU) of activity per gram of composition.

Suitable examples of proteases are the subtilisins which are obtained from particular strains of B. subtilis and B. licheniforms. Another suitable protease is obtained from a strain of Bacillus, having maximum activity throughout the pH range of 8-12, developed and sold by Novo Industries A/S under the registered trade name ESPERASE. The preparation of this enzyme and analogous enzymes is described in British Patent Specification No. 1,243,784 of Novo. Proteolytic enzymes suitable for removing protein-based stains that are commercially available include those sold under the tradenames ALCALASE and SAVINASE by Novo Industries A/S (Denmark) and MAXATASE by International Bio-Synthetics, Inc. (The Netherlands). Other proteases include Protease A (see EP 130,756) and Protease B (see European Patent Application Serial No. 87303761.8, filed April 28, 1987, and EP 130,756).

Most preferred is what is called herein "Protease C", which is a variant of an alkaline serine protease from Bacillus, particularly Bacillus lentus, in which arginine replaced lysine at position 27, tyrosine replaced valine at position 104, serine replaced asparagine at position 123, and alanine replaced threonine at position 274. Protease C is described in EP Application 90915958.4; U.S 5,185,250; and U.S 5,204,015. Also especially preferred are protease which are described in copending application U.S. Serial No. 08/136,797, entitled Protease-containing Cleaning Compositions and copending Application U.S. Serial No. 08/136,626, entitled Bleaching Compositions Comprising Protease Enzymes, which are incorporated herein by reference. Genetically modified variants, particularly of Protease C, are also included herein.

Amylases include, for example, α-amylases described in British Patent Specification No. 1,296,839 (Novo), RAPIDASE, International Bio-Synthetics, Inc. and TERMAMYL, Novo Industries.

The cellulase usable in the present invention include both bacterial or fungal cellulase. Preferably, they will have a pH optimum of between 5 and 9.5. Suitable cellulases are disclosed in U.S 4,435,307, which discloses fungal cellulase produced from Humicola insolens and Humicola strain DSM1800 or a cellulase 212-producing fungus belonging to the genus Aeromonas, and cellulase extracted from the hepatopancreas of a marine mollusk (Dolabella Auricula Solander). Suitable cellulases are also disclosed in GB-A-2.075.028; GB-A-2.095.275 and DE-OS-2.247.832. Cellulases such as CAREZYME (Novo) are especially useful, since they provide additional softening and appearance benefits to fabrics laundered in the present compositions.

Suitable lipase enzymes for detergent usage include those produced by microorganisms of the Pseudomonas group, such as Pseudomonas stutzeri ATCC 19.154, as disclosed in British Patent 1,372,034. See also lipases in Japanese Patent Application 53,20487, laid open to public inspection on February 24, 1978. This lipase is available from Amano Pharmaceutical Co. Ltd., Nagoya, Japan, under the trade name Lipase P "Amano," hereinafter referred to as "Amano-P." Other commercial lipases include Amano-CES, lipases ex Chromobacter viscosum, e.g. Chromobacter viscosum var. lipolyticum NRRLB 3673, commercially available from Toyo Jozo Co., Tagata, Japan; and further Chromobacter viscosum lipases from U.S. Biochemical Corp., U.S.A. and Disoynth Co., The Netherlands, and lipases ex Pseudomonas gladioli. The LIPOLASE enzyme derived from Humicola lanuginosa and commercially available from Novo (see also EP 341,947) is a preferred lipase for use herein.

Peroxidase enzymes are used in combination with oxygen sources, e.g., percarbonate, perborate, persulfate, hydrogen peroxide, etc. They are used for "solution bleaching," i.e. to prevent transfer of dyes or pigments removed from substrates during wash operations to other substrates in the wash solution. Peroxidase enzymes are known in the art, and include, for example, horseradish peroxidase, ligninase, and haloperoxidase such as chloro- and bromo-peroxidase. Peroxidase-containing detergent compositions are disclosed, for example, in PCT International Application WO 89/099813, published October 19, 1989, by O. Kirk, assigned to Novo Industries A/S. It may be desired to use, in combination with these peroxidases, materials viewed as being peroxidase accelerators such as phenolsulfonate and/or phenothiazine.

A wide range of enzyme materials and means for their incorporation into synthetic detergent compositions are also disclosed in U.S 3,553,139. Enzymes are further disclosed in U.S 4,101,457 and in U.S 4,507,219. Enzyme materials useful for liquid detergent formulations, and their incorporation into such formulations, are disclosed in U.S 4,261,868.

### Enzyme Stabilizers

A preferred optional ingredient for use in the present compositions is enzyme stabilizers. Enzymes for use in detergents can be stabilized by various techniques. Enzyme stabilization techniques are disclosed and exemplified in U.S 3,600,319 and EP 0 199 405, European Patent Application No. 86200586.5, published October 29, 1986, Venegas. Enzyme stabilization systems are also described, for example, in U.S 3,519,570. The enzymes employed herein can be stabilized by the presence of water-soluble sources of calcium and/or magnesium ions in the finished compositions which provide such ions to the enzymes. (Calcium ions are generally somewhat more effective than magnesium ions and are preferred herein if only one type of cation is being used.)

Additional stability can be provided by the presence of various other art-disclosed stabilizers, especially borate species: see Severson, U.S. 4,537,706. Typical detergents, especially liquids, will comprise from about 1 to about 30, preferably from about 2 to about 20, more preferably from about 5 to about 15, and most preferably from about 8 to about 12, millimoles of calcium ion per liter of finished composition. This can vary somewhat, depending on the amount of enzyme present and its response to the calcium or magnesium ions. The level of calcium or magnesium ions should be selected so that there is always some minimum level available for the enzyme, after allowing for complexation with builders, fatty acids, etc., in the composition. Any water-soluble calcium or magnesium salt can be used as the source of calcium or magnesium ions, including, but not limited to, calcium chloride, calcium sulfate, calcium malate, calcium maleate, calcium hydroxide, calcium formate, and calcium acetate, and the corresponding magnesium salts. A small amount of calcium ion, generally from about 0.05 to about 0.4 millimoles per liter, is often also present in the composition due to calcium in the enzyme slurry and formula water. In solid detergent compositions the formulation may include a sufficient quantity of a water-soluble calcium ion source to provide such amounts in the laundry liquor. In the alternative, natural water hardness may suffice.

It is to be understood that the foregoing levels of calcium and/or magnesium ions are sufficient to provide enzyme stability. More calcium and/or magnesium ions can be added to the compositions to provide an additional measure of grease removal performance. Accordingly, as a general proposition the compositions herein will typically comprise from about 0.05% to about 2% by weight of a water-soluble source of calcium or magnesium ions, or both. The amount can vary, of course, with the amount and type of enzyme employed in the composition.

The compositions herein may also optionally, but preferably, contain various additional stabilizers, especially borate-type stabilizers. Typically, such stabilizers will be used at levels in the compositions from about 0.25% to about 10%, preferably from about 0.5% to about 5%, more preferably from about 0.75% to about 3%, by weight of boric acid or other borate compound capable of forming boric acid in the composition (calculated on the basis of boric acid). Boric acid is preferred, although other compounds such as boric oxide, borax and other alkali metal borates (e.g., sodium ortho-, meta- and pyroborate, and sodium pentaborate) are suitable. Substituted boric acids (e.g., phenylboronic acid, butane boronic acid, and p-bromo phenylboronic acid) can also be used in place of boric acid. It is to be recognized that such materials may also be used in formulations as the sole stabilizer as well as being used in combination with added calcium and/or magnesium ions.

Finally, it may be desired to add chlorine scavengers, especially to protease-containing compositions, to protect the enzymes from chlorine typically present in municipal water supplies. Such materials are described, for example, in U.S. Patent 4,810,413 to Pancheri et al.

Various other optional adjunct ingredients may also be used to provide fully-formulated detergent compositions. The following ingredients are described for the convenience of the formulator, but are not intended to be limiting thereof.

Other preferred optional ingredients include polymeric soil release agents, materials effective for inhibiting the transfer of dyes from one fabric to another during the cleaning process (i.e., dye transfer inhibiting agents), polymeric dispersing agents, suds suppressors, optical brighteners or other brightening or whitening agents, chelating agents, fabric softening clay, anti-static agents, other active ingredients, stabilizers, such as well known antioxidants and reductive agents, emulsifiers, bacteriocides, colorants, perfumes, preservatives, anti ionisation agents, antifoam agents,
carriers, hydrotropes, processing aids, dyes or pigments, solvents for liquid formulations, solid fillers for bar compositions, etc.

Liquid detergent compositions can contain water and other solvents as carriers. Low molecular weight primary or secondary alcohols exemplified by methanol, ethanol, propanol, and isopropanol are suitable. Monohydric alcohols are preferred for solubilizing surfactant, but polyols such as those containing from 2 to about 6 carbon atoms and from 2 to about 6 hydroxy groups (e.g., 1,3-propanediol, ethylene glycol, glycerine, and 1,2-propanediol) can also be used.

The compositions may contain from 5% to 90%, typically 10% to 50% of such carriers.

Granular detergents can be prepared, for example, by spray-drying (final product density about 520 g/l) or agglomerating (final product density above about 600 g/l) the Base Granule. The remaining dry ingredients can then be admixed in granular or powder form with the Base Granule, for example in a rotary mixing drum, and the liquid ingredients (e.g., nonionic surfactant and perfume) can be sprayed on.

A further aspect of the invention is a method for releasing an active alcohol component, preferably a perfume alcohol component, which comprises contacting a composition according to the invention with a material (e.g water), so that at least one of the nitrogen atom of said beta-amino ester compound is protonated.

Not to be bound by theory, it is believed that such protonation is obtained upon a consecutive pH drop of the environment and/or upon a consecutive high dilution of said environment. The pH drop may occur by any suitable means. This can be done by contacting said composition with water upon a rinsing or dilution step and wherein the decrease of the pH measured in the environment is of at least 0.5 unit versus the original pH of the composition.

High dilution of at least a factor 10 is also believed to result in the protonation of at least one of the nitrogen atom of said beta-amino ester compound.

Rinsing or dilution steps are those which commonly occur in a hard surface cleaning process or dishwashing process whereby the composition of the invention is applied in its neat form or in diluted form onto said surfaces, then left to act onto said surfaces and then removed by rinsing. Of course, if suitable, the rinsing step may be rendered optional where the composition of the invention is in diluted form.

In a laundry washing process such as a hand wash or a washing machine process, the composition of the invention is applied in its neat form or in diluted form onto said fabrics, then left to act onto said fabrics and then removed by rinsing.

The invention is illustrated in the following non limiting examples, in which all percentages are on a weight basis unless otherwise stated.

In the compositions, the abbreviated component identifications have the following meanings:
- LAS :: Sodium linear C12 alkyl benzene sulphonate
- TAS :: Sodium tallow alcohol sulphate
- XYAS :: Sodium C_{1X} - C_{1Y} alkyl sulfate
- XYEZ :: A C₁ₓ - C_{1y} predominantly linear primary alcohol condensed with an average of Z moles of ethylene oxide
- Soap :: Sodium linear alkyl carboxylate derived from an 80/20 mixture of tallow and a coconut oils.
- NaSKS-6 :: Crystalline layered silicate of formula δ-Na₂Si₂O₅
- MA/AA :: Copolymer of 1:4 maleic/acrylic acid, average molecular weight about 70,000.
- STPP :: Anhydrous sodium tripolyphosphate
- Zeolite A :: Hydrated Sodium Aluminosilicate of formula Na₁₂(A1O₂SiO₂)₁₂. 27H₂O having a primary particle size in the range from 1 to 10 micrometers
- Percarbonate :: Anhydrous sodium percarbonate bleach coated with a coating of sodium silicate (Si₂O:Na₂O ratio = 2:1) at a weight ratio of percarbonate to sodium silicate of 39:1
- PB1 :: Anhydrous sodium perborate bleach of nominal formula NaBO₂.H₂O₂
- PB4 :: Sodium perborate tetrahydrate of nominal formula NaBO₂.3H₂O.H₂O₂
- Protease :: Proteolytic enzyme sold under the tradename Savinase by Novo Industries A/S with an activity of 13 KNPU/g.
- Protease # :: Proteolytic enzyme sold under the tradename Savinase by Novo Industries A/S with an activity of 4 KNPU/g.
- Amylase :: Amylolytic enzyme sold under the tradename Termamyl 60T by Novo Industries A/S with an activity of 300 KNU/g
- Carezyme :: Cellulosic enzyme sold by Novo Industries A/S with an activity of 1000 CEVU/g
- Lipase :: Lipolytic enzyme sold under the tradename Lipolase by Novo Industries A/S with an activity of 165 KLU/g
- CMC :: Sodium carboxymethyl cellulose
- DETPMP :: Diethylene triamine penta (Methylene phosphonic acid), marketed by Monsanto under the Tradename Dequest 2060
- HEDP :: Hydroxy-ethane 1,1 diphosphonic acid
- SRA (Soil Release : Agents): Sulfobenzoyl end capped esters with oxyethylene oxy and terephthaloyl backbone
- Sulphate :: Anhydrous sodium sulphate
- Brightener 1 :: Disodium 4,4'-bis(2-Sulphostyryl) biphenyl
- Brightener 2 :: Disodium 4,4'-bis(4-anilino-6-morpholino-1.3.5-triazin-2-yl)amino) stilbene-2:2'-disulphonate.
- Photoactivated bleach :: Sulphonated Zinc Phthalocyanine encapsulated in dextrin soluble polymer
- Silicone antifoam :: Polydimethylsiloxane foam controller with Siloxane-oxyalkylene copolymer as dispersing agent with a ratio of said foam controller to said dispersing agent of 10:1 to 100:1.
- Nonionic :: C₁₃-C₁₅ mixed ethoxylated/propoxylated fatty alcohol with an average degree of ethoxylation of 3.8 and an average degree of propoxylation of 4.5 sold under the tradename Plurafac LF404 by BASF Gmbh (low foaming)
- Metasilicate :: Sodium metasilicate (SiO₂:Na₂O ratio = 1.0)
- Silicate :: Amorphous Sodium Silicate (SiO₂:Na₂O ratio = 2.0)
- Carbonate :: Anhydrous sodium carbonate
- 480N :: Random copolymer of 3:7 acrylic/methacrylic acid, average molecular weight about 3,500
- Citrate :: Tri-sodium citrate dihydrate
- TAED :: Tetraacetyl ethylene diamine
- Cationic precursor: Cationic peroxyacid bleach precursor salt of trialkyl ammonium methylene C₅-alkyl caprolactam with tosylate
- BzP :: Dibenzoyl peroxide
- PMT :: 1-phenyl-5-mercapto-1,2,3,4-tetrazole
- Bismuth nitrate :: Bismuth nitrate salt
- Paraffin :: Paraffin oil sold under the tradename Winog 70 by Wintershall.
- BD/MA :: Copolymer of butadiene/maleic acid as sold by Polysciences inc under the tradename reference no. 07787
- BSA :: Amylolytic enzyme sold under the tradename LE17 by Novo Industries A/S (approx 1% enzyme activity)
- DGAA :: N,N-di(geranyl acetate)-N-dodecylamine
- MGAA :: Geranyl N-octylaminoacetate

### Synthesis of geranyl bromoacetate of formula:

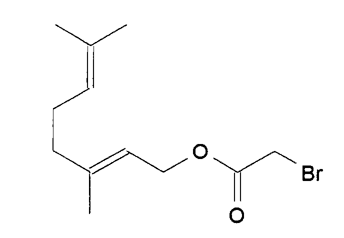

Bromoacetyl bromide (0.055 mol, 4.74 ml) was mixed with dichloromethane (80 ml), in a 150 ml conical flask, cooled on a cold water-bath. To this solution were added dropwise a mixture of geraniol (0.055 mol, 9.6 ml), pyridine (0.055 mol, 4.4 ml) in dichloromethane (20 ml), the total addition time being about 15-30 minutes. The dropping funnel was fitted with a calcium chlorure drying tube. The reaction mixture was left to stirr over the cold water-bath for five hours. Then, the white precipitate of pyridinium chlorure was filtered off and the reaction mixture was washed with distilled water (3x150 ml), dried over MgSO₄ and the solvent removed under reduced pressure, yielding geranyl bromoacetate as a dark brown oil (14.2 g, 88.5%)
C₁₂H₁₉BrO₂ M= 275.19 g/mol
IR: n C=O 1762 and 1737 cm⁻¹, n C=C 1669 cm⁻¹ and 1637 cm⁻¹, n C-O ester 1281 cm⁻¹, n C-Br 675 cm⁻¹,
dH: (270 MHz, CDCl₃) 1.60 (CH₃-C=, s, 3H), 1.68 and 1.72 (CH₃-C=, s, 6H), 2.07 (=CH-CH₂, =C(CH₃)-CH₂, m, 4H), 3.84 (CH₂OOCCH₂Br, s, 2H), 4.68 (=CH-CH₂-OOC, d, J 7Hz, 2H), 5.07 ((CH₃)₂C=CH, t, J 5.5 Hz, 1H), 5.35 (=CH-CH₂OOC, t, J 7 Hz, 1H)
dC/DEPT: (70 MHz, CDCl₃) 16.4 17.5 25.5 CH₃-C=, 25.9 CH₂OOCCH₂Br, 26.1 =CH-CH₂-CH₂, 39.4 =C(CH₃)-CH₂, 62.9 =CH-CH₂-OOC, 117.3 =CH-CH₂OOC, 123.5 (CH₃)₂C=CH, 131.7 =C(CH₃)₂, 143.3 =C(CH₃)-CH₂, 167.0 C=O
H-C COSY:
   Direct coupling of the three singlets at 1.60 1.68 and 1.72 ppm (1H) with 16.3 17.5 and 25.5 ppm (13C)
   Direct coupling of the massif at 2.07 ppm (1H) with 26.1 and 39.3 ppm (13C)
   Direct coupling of the singulet at 3.84 ppm (1H) with 25.9 ppm (13C)
   Direct coupling of the doublet at 4.68 ppm (1H) with 62.9 ppm (13C)
   Direct coupling of the massif at 5.07 ppm (1H) with 123.5 ppm (13C)
   Direct coupling of the triplet at 5.32 ppm (1H) with 117.3 ppm (13C)

### Example 1

### Synthesis of geranyl N-dodecylaminoacetate (75) of formula:

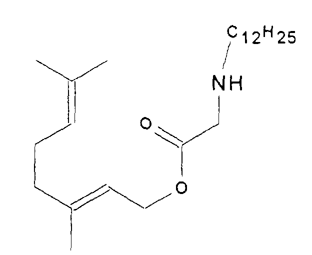

The above compound was synthesised by three different experiments:

### First experiment

Dodecylamine (18.4 ml, 80 mmol, 2 eq) in acetone (20 ml) was slowly added to geranyl bromoacetate (11.01 g, 40 mmol, 1 eq), in acetone (60 ml), at ambient temperature. After two hours stirring, the reaction seemed completed by 1H NMR. A solution of hydrochloric acid 5N in isopropanol (1 ml, 0.005 mol, ∼0.12 eq) was then added to protonate any remaining dodecylamine. After 5 minutes stirring, the acetone was evaporated under vacuum and ether was added (100 ml). The precipitate of dodecylamine bromhydride obtained was filtered off and the filtered solution was placed at -18°C for 18 hours. Thereafter, the said solution was again filtered before the ether was evaporated under vacuum conditions. The geranyl bromoacetate was converted at ∼85% in N,N-di(geranyl acetate)-N-dodecylamine (two geranyl chains per molecule) and at only 15% in geranyl N-dodecylaminoacetate. In mol, the final reactional mixture contained ∼75% N,N-di(geranyl acetate)-N-dodecylamine, ∼25% de geranyl N-dodecylaminoacetate and less than 5% of geranyl bromoacetate.

### Second experiment

Geranyl bromoacetate (2.02 g, 8 mmol, 1 eq), in acetone (40 ml), was slowly added to dodecylamine (6.4 ml, 28 mmol, 3.5 eq) , in acetone (40 ml), at ambient temperature. After two hours stirring, the reaction seemed completed by 1H NMR. The geranyl bromoacetate was converted at 40% in N,N-di(geranyl acetate)-N-dodecylamine (two geranyl chains per molecule) and at 60% in geranyl dodecylaminoacetate. In mole, the final reactional mixture contained ∼25% N-dodecylamine N,N-di(geranyl acetate), ∼75% geranyl N-dodecylaminoacetate and less than 5% geranyl bromoacetate.

When the reaction was made with 5 equivalents of dodecylamine instead of 3.5, the geranyl bromoacetate was converted at ∼30% in N,N-di(geranyl acetate)-N-dodecylamine (two geranyl chains per molecule) and at 70% in geranyl dodecylaminoacetate. In mol, the final reactional mixture contained ∼15-20% N,N-di(geranyl acetate)-N-dodecylamine, 80-85% geranyl N-dodecylaminoacetate and less than 5% geranyl bromoacetate.

Both samples were worked-up as for example 1

### Third experiment

Geranyl bromoacetate (2.02 g, 8 mmol, 1 eq), in toluene (40 ml), was slowly added to dodecylamine (6.4 ml, 28 mmol, 3.5 eq), in toluene (40 ml), at ambient temperature. After seven hours stirring, the reaction seemed completed by 1H NMR. The geranyl bromoacetate was converted at over 90% in geranyl N-dodecylaminoacetate. The proportion of N,N-di(geranyl acetate)-N-dodecylamine and remaining geranyl bromoacetate were less than 10%. Chloroform and ethyl acetate are two other solvents leading to a similar selective formation of geranyl N-dodecylaminoacetate.

All samples were worked-up as for example 1

### Geranyl N-Dodecylaminoacetate (75)

C₂₄H₄₅NO₂ M= 379.63 g/mol
dH/ H-H COSY: (270 MHz, CDCl₃) 0.88 (CH₃CH₂, t, J 6.6 Hz, 3H), 1.26 (CH₂, m, 18H), 1.47 (CH₂CH₂N, m, 2H), 1.60 1.68 1.71 (CH₃-C=, three s 9H), 2.07 (=CH-CH₂, =C(CH₃)-CH₂, m, 4H), 2.59 (CH₂CH₂N, t, 7.1 Hz, 2H), 3.40 (CH₂OOCCH₂NHCH₂, s, 2H), 4.65 (=CH-CH₂-OOC, d, J 6.9 Hz, 2H), 5.08 (=CH, m, 1H), 5.34 (=CH-CH₂OOC, t, J 7.1 Hz, 1H)
dC/DEPT H-C COSY: (70 MHz, CDCl₃) 14.0 CH₃CH₂, 16.3 17.5 25.5 CH₃-C=, 22.6 27.1 29.2 29.43 29.52 31.8 CH₂, 26.2 =CH-CH₂-CH₂, 27.5 CH₂CH₂N, 39.4 =C(CH₃)-CH₂, 49.6 CH₂CH₂N, 50.9 CH₂OOCCH₂NCH₂, 61.4 =CH-CH₂-OOC, 117.9 =CH-CH₂-OOC, 123.4 (CH₃)₂C=CH, 132.0 =C(CH₃)₂, 142.4 =C(CH₃)-CH₂, 172.5 C=O
H-C COSY:
   Direct coupling of the triplet at 0.88 ppm (1H) with 14.0 ppm (13C)
   Direct coupling of the massif at 1.26 ppm (1H) with 22.6 27.1 29.2 29.43 29.52 31.8 ppm (13C)
   Direct coupling of the three singlets at 1.60 1.68 and 1.71 ppm (1H) with 16.3 17.5 and 25.5 ppm (13C)
   Direct coupling of the massif at 1.47 ppm (1H) with 27.5 ppm (13C)
   Direct coupling of the massif at 2.07 ppm (1H) with 26.2 and 39.4 ppm (13C)
   Direct coupling of the triplet at 2.59 ppm (1H) with 49.6 ppm (13C)
   Direct coupling of the singlet at 3.40 ppm (1H) with 50.9 ppm (13C)
   Direct coupling of the doublet at 4.65 ppm (1H) with 61.4 ppm (13C)
   Direct coupling of the massif at 5.08 ppm (1H) with 123.4 ppm (13C)
   Direct coupling of the triplet at 5.34 ppm (1H) with 117.9 ppm (13C)
SM (electrospray) MH⁺= C₂₄H₄₆NO₂= 380.64 g/mol
   Found: m/z = 380.5

### N,N-di(geranyl acetate)-N-dodecylamine

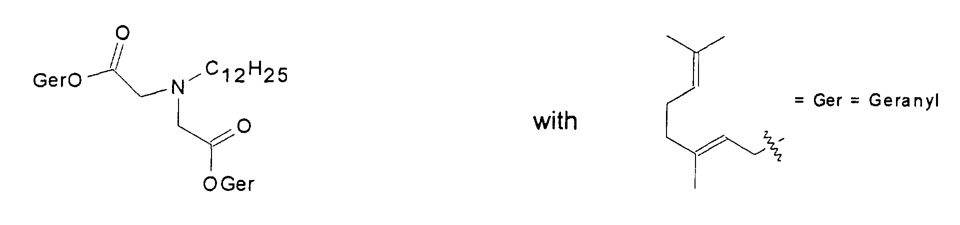

Dodecylamine (18.4 ml, 80 mmol, 1 eq) in acetone (50 ml) was slowly added, at room temperature, to a solution of geranyl bromoacetate (46.24 g, 168 mmol, 2.1 eq) and diisopropylamine (22 ml, 168 mmol, 2.1 eq), in acetone (200 ml). The reaction progress was followed by 1H NMR. After 10 hours stirring, the reaction seemed completed by NMR. The diisopropylamine bromohydride formed was filtered off and the acetone was evaporated under vacuum and petroleum ether 40-60°C (100 ml) was added. This led to the precipitation of more diisopropylamine bromohydride which was filtered before the petroleum ether 40-60°C is evaporated under vacuum. This last set of operation was repeated three times, until no more diisopropylamine bromohydride precipitates. The composition of the final oil(46.5 g) was (in mol) ∼90% N,N-di(geranyl acetate)-N-dodecylamine, ∼5% geranyl bromoacetate and ∼5% geranyl (N,N-diisopropylamino)acetate.
C₃₆H₆₃NO₄ M= 573.90 g/mol
dH/ H-H COSY: (270 MHz, CDCl₃) 0.88 (CH₃CH₂, t, J 6.6 Hz, 3H), 1.26 (CH₂, m, 18H), 1.47 (CH₂CH₂N, m, 2H), 1.60 1.67 1.70 (CH₃-C=, three s 18H), 2.06 (=CH-CH₂, =C(CH₃)-CH₂, m, 8H), 2.69 (CH₂CH₂N, t, 7.6 Hz, 2H), 3.55 (CH₂OOCCH₂NHCH₂, s, 4H), 4.62 (=CH-CH₂-OOC, d, J 7.3 Hz, 4H), 5.08 (=CH, m, 2H), 5.34 (=CH-CH₂OOC, t, J 6.6 Hz, 2H)
H-H COSY:
   Coupling of the triplet at 0.88 ppm and the massif at 1.26 ppm
   Coupling of the massif at 1.47 ppm and the triplet at 2.69 ppm
   Coupling of the singlets at 1.60 1.67 and 1.70 ppm and the massif at 2.06 ppm as well as the two signals at 5.08 and 5.34 ppm
   Coupling of the massif at 2.06 ppm and the one at 5.08 ppm
   Coupling of the doublet at 4.62 ppm and the triplet at 5.34 ppm
dC/DEPT H-C COSY: (70 MHz, CDCl₃) 13.8 CH₃CH₂, 16.1 17.3 25.3 CH₃-C=, 22.4 26.9 29.07 29.25 29.34 31.6 CH₂, 26.0 =CH-CH₂-CH₂, 27.6 CH₂CH₂N, 39.2 =C(CH₃)-CH₂, 54.1 CH₂CH₂N, 54.8 CH₂OOCCH₂NCH₂, 60.9 =CH-CH₂-OOC, 118.0 =CH-CH₂-OOC, 123.4 (CH₃)₂C=CH, 131.3 =C(CH₃)₂, 141.9 =C(CH₃)-CH₂, 170.9 C=O
H-C COSY:
   Direct coupling of the triplet at 0.88 ppm (1H) with 13.8 ppm (13C)
   Coupling of the massif at 1.26 ppm (1H) with 22.4 26.9 29.25 29.34 31.6 ppm (13C)
   Coupling of the three singlets at 1.60 1.67 and 1.70 ppm (1H) with 16.1 17.3 and 25.3 ppm (13C)
   Direct coupling of the massif at 1.47 ppm (1H) with 27.6 ppm (13C)
   Direct coupling of the massif at 2.06 ppm (1H) with 26.0 and 39.2 ppm (13C)
   Direct coupling of the triplet at 2.69 ppm (1H) with 54.1 ppm (13C)
   Direct coupling of the singlet at 3.55 ppm (1H) with 54.8 ppm (13C)
   Direct coupling of the doublet at 4.62 ppm (1H) with 60.9 ppm (13C)
   Direct coupling of the massif at 5.08 ppm (1H) with 123.4 ppm (13C)
   Direct coupling of the triplet at 5.34 ppm (1H) with 118.0 ppm (13C)
SM (electrospray) MH⁺= C₃₆H₆₄NO₄⁺= 574.91 g/mol
   Found: m/z = 574.5

### Synthesis of the 1,3-diaminopropane-N,N,N',N'-tetra(geranyl acetate)

Geranyl bromoacetate (3.03 g, 22 mmol, 4.4 eq) and diisopropylamine (2.45 ml, 22 mmol, 4.4 eq) were mixed with 1,3-diaminopropane (0.42 ml, 5 mmol, 1 eq), in acetone (40 ml), at room temperature. The reaction progress was followed by 1H NMR. After 5 hours stirring, the reaction seemed completed by NMR. The diisopropylamine bromohydride formed was filtered off and the acetone was evaporated under vacuum. The brown oil obtained was redisolved in acetone (50 ml), filtered and the acetone was evaporated under vacuum. This last set of operation was repeated three times with petroleum ether 60-80°C, until no more diisopropylamine bromohydride precipitates. The composition of the final oil was :

| Composition of the final oil | % by 1H NMR |
|---|---|
| 1,3-diaminopropane-N,N,N',N'-tetra(geranyl acetate) | ∼90% |
| Geranyl bromoacetate | 5-10% |
| Diisopropylamine bromohydride | ∼2% |
| Diisopropylamine | <<1% |
| Geranyl (N,N-diisopropylamino)acetate | ∼2% |

### 1,3-diaminopropane-N,N,N',N'-tetra(geranyl acetate)

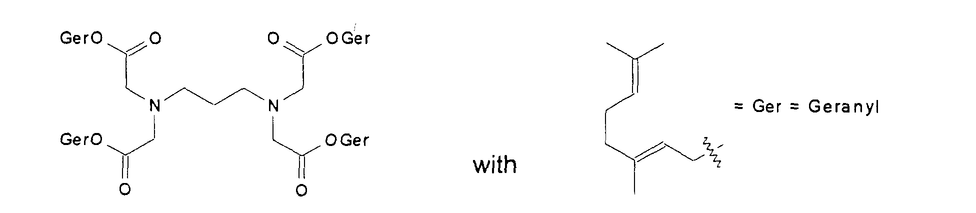

C₅₁H₈₂N₂O₈ M = 851.22 g/mol
dH/ H-H COSY: (270 MHz, CDCl₃) 1.50-1.80 (CH₂CH₂N, m, 2H and 1.60 1.68 1.70 CH₃-C=, three s 3*12H, in total 38H), 2.07 (=CH-CH₂, =C(CH₃)-CH₂, m, 16H), 2.76 (CH₂CH₂N, t, 7.3 Hz, 4H), 3.55 (CH₂OOCCH₂NHCH₂, s, 8H), 4.61 (=CH-CH₂-OOC, d, J 7.3 Hz, 8H), 5.08 (=CH, m, 4H), 5.33 (=CH-CH₂OOC, t, J 7.6 Hz, 4H)
H-H COSY:
   Coupling of the massif at 1.5-1.8 ppm and the signals at 2.07, 2.76, 4.61, 5.08 and 5.33 ppm
   Coupling of the massif at 2.07 ppm and the one at 5.08 ppm
   Coupling of the triplet at 2.76 ppm and the massif at 1.5-1.8 ppm
   Coupling of the doublet at 4.61 ppm and the triplet at 5.33 ppm
dC/DEPT H-C COSY: (70 MHz, CDCl₃) 16.5 17.7 25.7 CH₃-C=, 23.5 CH₂CH₂N, 26.3 =CH-CH₂-CH₂, 39.5 =C(CH₃)-CH₂, 52.0 CH₂CH₂N, 54.9 CH₂OOCCH₂NCH₂, 61.3 =CH-CH₂-OOC, 118.1 =CH-CH₂-OOC, 123.7 (CH₃)₂C=CH, 131.8 =C(CH₃)₂, 142.4 =C(CH₃)-CH₂, 171.3 C=O
H-C COSY:
   Direct coupling of the massif at 1.5-1.8 ppm (1H) with 23.5 and 16.5, 17.7 and 25.7 ppm (13C)
   Direct coupling of the massif at 2.07 ppm (1H) with 26.3 and 39.5 ppm (13C)
   Direct coupling of the triplet at 2.76 ppm (1H) with 52.0 ppm (13C)
   Direct coupling of the singlet at 3.55 ppm (1H) with 54.9 ppm (13C)
   Direct coupling of the doublet at 4.61 ppm (1H) with 61.3 ppm (13C)
   Direct coupling of the massif at 5.08 ppm (1H) with 123.7 ppm (13C)
   Direct coupling of the triplet at 5.33 ppm (1H) with 118.1 ppm (13C).

### Example 2

The following hard surface cleaning compositions A to C according to the invention were prepared.

| | A | B | C |
|---|---|---|---|
| Dobanol 23-3® | 3.20 | 3.20 | 1.28 |
| Lutensol AO30® | 4.80 | 4.80 | 1.92 |
| Dobanol C7-11EO6® | 8.0 | 8.0 | 3.20 |
| Topped Palm Kernal Fat acid, Na salt | 0.80 | 0.80 | 0.40 |
| C8 Alkyl sulphate, Na salt | 2.0 | 2.0 | 0.8 |
| Parafin sulphonate, Na salt | 3.0 | 3.0 | 1.20 |
| Cumene sulphonate, Na salt | 3.0 | 3.0 | 1.20 |
| Perfume | 0.8 | 0.8 | 0.6 |
| Branched alcohol, Isofol 16® | - | - | 0.30 |
| DGAA | 1.86 | - | 0.6 |
| MGAA | - | 2.49 | - |
| NaOH up to | pH 10 | pH 10 | pH 10 |
| Water and Minors up to 100% | | | |

Processing of composition A and B were made by adding all materials and mixing together with the aminoacetate component.

Processing of composition C was made by addition of all components to a premix of DGAA and perfume followed by addition of the remaining water. Alternatively all materials except DGAA and perfume were mixed, followed by the addition of DGAA and heating to 70°C for a short period of time and the mixture is thereafter left to cool at ambient temperature (20°C). Once the mixture was cooled to ambient temperature, the perfume component was added with stirring.

### Example 3

The following dishwashing machine compositions according to the invention were prepared.

| | D | E | F | G | H | I | J |
|---|---|---|---|---|---|---|---|
| Citrate | 15.0 | 15.0 | 15.0 | 15.0 | 15.0 | 15.0 | - |
| 480N | 6.0 | 6.0 | 6.0 | 6.0 | 6.0 | 6.0 | - |
| Carbonate | 17.5 | 17.5 | 17.5 | 17.5 | 17.5 | 17.5 | - |
| STPP | - | - | - | - | - | - | 38.0 |
| Silicate (as SiO₂) | 8.0 | 8.0 | 8.0 | 8.0 | 8.0 | 8.0 | 14.0 |
| Metasilicate (as SiO₂) | 1.2 | 1.2 | 1.2 | 1.2 | 1.2 | 1.2 | 2.5 |
| PB1 (AvO) | 1.2 | 1.2 | 1.5 | 1.5 | 1.5 | 2.2 | 1.2 |
| TAED | 2.2 | 2.2 | 2.2 | - | - | 2.2 | 2.2 |
| BzP | - | - | - | 0.8 | - | - | - |
| Cationic precursor | - | - | - | - | 3.3 | - | - |
| Paraffin | 0.5 | 0.5 | 0.5 | 0.5 | 0.5 | 0.5 | 0.5 |
| Bismuth nitrate | - | 0.2 | 0.2 | 0.2 | 0.3 | 0.4 | 0.2 |
| BD/MA | - | - | - | - | - | - | 0.5 |
| PMT | - | - | - | - | - | - | 0.5 |
| Protease | 0.04 | 0.04 | 0.04 | 0.04 | 0.04 | 0.04 | 0.04 |
| Amylase | 0.03 | 0.03 | 0.03 | 0.03 | 0.03 | 0.03 | - |
| BSA | - | - | - | - | - | - | 0.03 |
| DETPMP | 0.13 | 0.13 | 0.13 | 0.13 | 0.13 | 0.13 | - |
| HEDP | 1.0 | 1.0 | 1.0 | 1.0 | 1.0 | 1.0 | - |
| Nonionic | 2.0 | 2.0 | 2.0 | 2.0 | 2.0 | 2.0 | 1.5 |
| MGAA | 0.5 | 0.5 | 0.5 | 1.86 | 1.86 | 1.86 | 1.86 |
| Sulphate | 23.0 | 22.8 | 22.4 | 22.7 | 22.2 | 21.5 | 0.3 |
| misc inc Moisture to balance | | | | | | | |
| pH (Measured as a 1% solution in distilled water at 20°C) | 10.7 | 10.7 | 10.7 | 10.7 | 10.7 | 10.7 | 11.0 |

### Example 4

The following laundry compositions according to the invention were prepared.

| | **K** | **L** | **M** |
|---|---|---|---|
| Blown Powder | | | |
| STPP | 24.0 | - | 24.0 |
| Zeolite A | - | 24.0 | - |
| Sulphate | 9.0 | 6.0 | 13.0 |
| MA/AA | 2.0 | 4.0 | 2.0 |
| LAS | 6.0 | 8.0 | 11.0 |
| TAS | 2.0 | - | - |
| Silicate | 7.0 | 3.0 | 3.0 |
| CMC | 1.0 | 1.0 | 0.5 |
| Brightener 2 | 0.2 | 0.2 | 0.2 |
| Soap | 1.0 | 1.0 | 1.0 |
| DETPMP | 0.4 | 0.4 | 0.2 |

| Spray On | | | |
|---|---|---|---|
| 45E7 | 2.5 | 2.5 | 2.0 |
| 25E3 | 2.5 | 2.5 | 2.0 |
| Silicone antifoam | 0.3 | 0.3 | 0.3 |
| Perfume | 0.3 | 0.3 | 0.3 |
| MGAA | 1.86 | 0.5 | 1.86 |

| Dry additives | | | |
|---|---|---|---|
| Carbonate | 6.0 | 13.0 | 15.0 |
| PB4 | 18.0 | 18.0 | 10 |
| PB1 | 4.0 | 4.0 | - |
| TAED | 3.0 | 3.0 | 1.0 |
| Photoactivated bleach | 0.02% | 0.02% | 0.02% |
| Protease # | 1.0 | 1.0 | 1.0 |
| Lipolase | 0.4 | 0.4 | 0.4 |
| Termamyl | 0.25 | 0.30 | 0.15 |
| Sulphate | 3.0 | 3.0 | 5.0 |
| Balance (Moisture & Miscellaneous) | 100.0 | 100.0 | 100.0 |

| | **N** | **O** |
|---|---|---|
| Agglomerate | | |
| 45AS | 11.0 | 14.0 |
| Zeolite A | 15.0 | 6.0 |
| Carbonate | 4.0 | 8.0 |
| MA/AA | 4.0 | 2.0 |
| CMC | 0.5 | 0.5 |
| DETPMP | 0.4 | 0.4 |
| | | |

| Spray On | | |
|---|---|---|
| 25E5 | 5.0 | 5.0 |
| Perfume | 0.5 | 0.5 |
| MGAA | 0.5 | 0.5 |

| Dry Additives | | |
|---|---|---|
| HEDP | 0.5 | 0.3 |
| SKS 6 | 13.0 | 10.0 |
| Citrate | 3.0 | 1.0 |
| TAED (4) | 5.0 | 7.0 |
| Percarbonate | 20.0 | 20.0 |
| SRA | 0.3 | 0.3 |
| Protease # | 1.4 | 1.4 |
| Lipolase | 0.4 | 0.4 |
| Carezyme | 0.6 | 0.6 |
| Termamyl | 0.6 | 0.6 |
| Silicone antifoam particle | 5.0 | 5.0 |
| Brightener 1 | 0.2 | 0.2 |
| Brightener 2 | 0.2 | - |
| Balance (Moisture and Miscellaneous) | 100 | 100 |
| Density (g/litre) | 850 | 850 |

## Claims

1. A compound of formula: 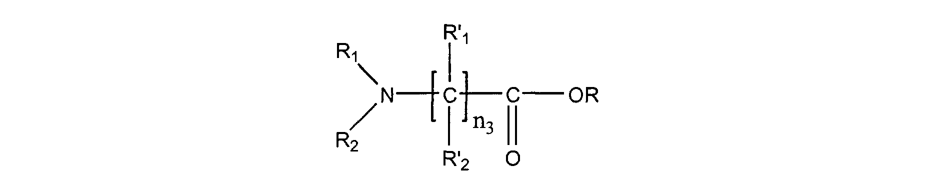 wherein each R'₁, R'₂ is hydrogen, and
wherein each R₁, R₂, independently, is selected from hydrogen, alkyl group, aryl group, 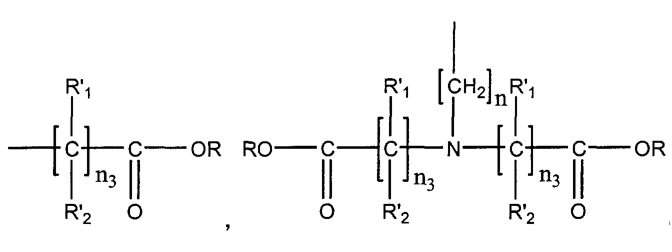 and 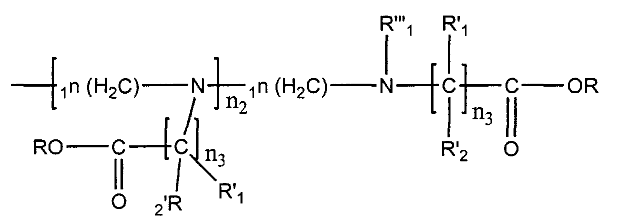
wherein each R'''₁ is selected from hydrogen, alkyl group, aryl group and
wherein each n, n₁, independently is an integer lying in the range from 1 to 20, and
wherein each n₂ is an integer lying in the range of 1 to 6,
wherein each n₃, independently, is an integer lying in the range from 1 to 3, and
wherein each R, independently, is an organic chain of an active alcohol component, said active alcohol being selected from flavour alcohols and perfume alcohols; with the proviso that at least one of R₁ and/or R₂ is: 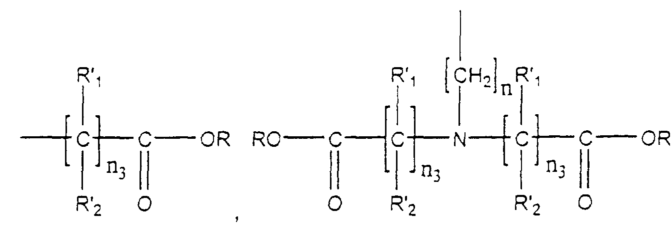
or 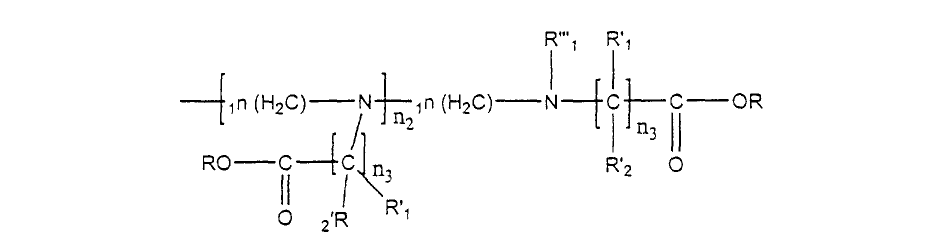 and that the compound is not:

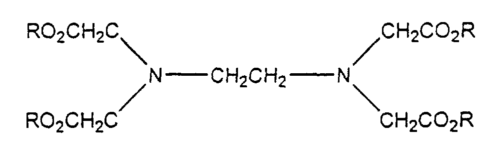

2. A compound according to Claim 1, wherein each R₁ is a hydrogen and R₂ is

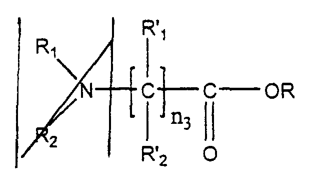

3. A compound according to Claim 1, wherein each R₁, R₂ is

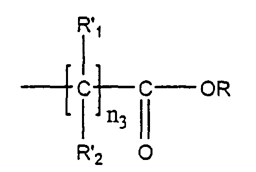

4. A compound according to Claim 1, wherein each R₁ is 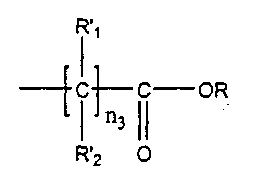 and R₂ is

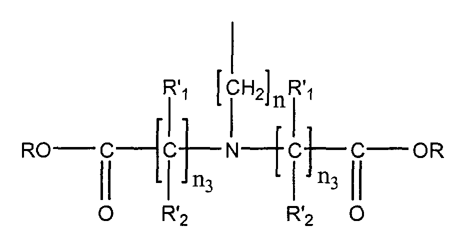

5. A compound according to Claim 1, wherein each R₁, R₂ is

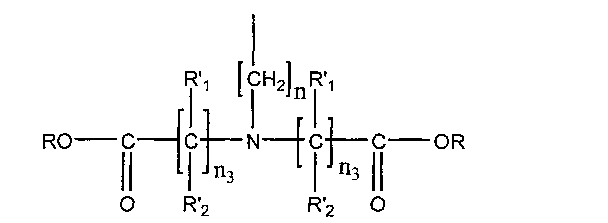

6. A compound according to Claim 1, wherein R₁ is: 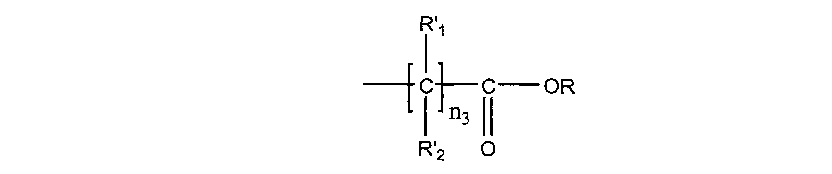 and R₂ is

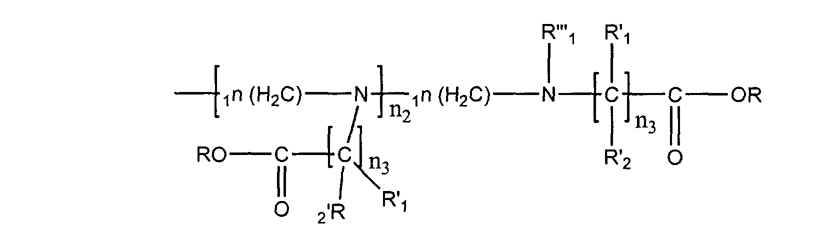

7. A compound according to Claim 1, wherein R₁ and R₂ are:

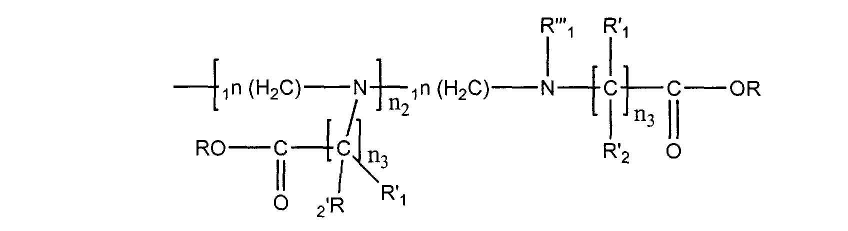

8. A compound according to any one of Claims 1-7, wherein said R group is the organic chain of a perfume alcohol.

9. A compound according to Claim 8 wherein said R group is the organic chain of a perfume alcohol, said alcohol being selected from 2-phenoxyethanol, phenylethylalcohol, geraniol, citronellol, 3-methyl-5-phenyl-1-pentanol, 2,4-dimethyl-3-cyclohexane-1-methanol, linalool, tetrahydrolinalool, 1,2-dihydromyrcenol, hydroxycitronellal, farnesol, menthol, eugenol, vanilin, cis-3-hexanol, terpineol and mixtures thereof, preferably geraniol and/or citronellol.

10. A process for preparing a compound as defined in any one of Claims 1-9 which comprises the steps of:
(a) esterifying the alcohol into its halogenoacetate ester form in presence of an amine, and
(b) reacting said halogenoacetate ester with the desired amine in presence of a sterically hindered amine.

11. Use of a compound according to any of Claims 1-9 as a precursor of the corresponding betaine ester compound.

12. A laundry and cleaning composition comprising one or more surfactant components and incorporating a compound according to any of Claims 1-9, wherein said composition is alkaline.

13. A composition according to Claim 12 wherein said composition is selected from a detergent composition, a hard surface cleaning composition and a dish-washing composition.

14. A composition according to Claim 12 wherein said composition is a hard surface cleaning composition.

15. A method for releasing an active alcohol component which comprises contacting a composition as defined in Claim 12 with a material so that at least one of the nitrogen atom of the beta-amino ester is protonated.

16. A method according to Claim 15 wherein said material is water.

17. A method according to either of Claims 15 or 16 wherein said active component is a perfume alcohol component.

## Patentansprüche

1. Verbindung der Formel: 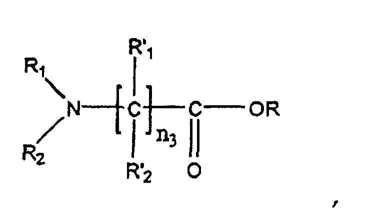 worin jeder Rest R'₁, R'₂ Wasserstoff darstellt, und
worin jeder Rest R₁, R₂ unabhängig unter Wasserstoff, einer Alkylgruppe, einer Arylgruppe, 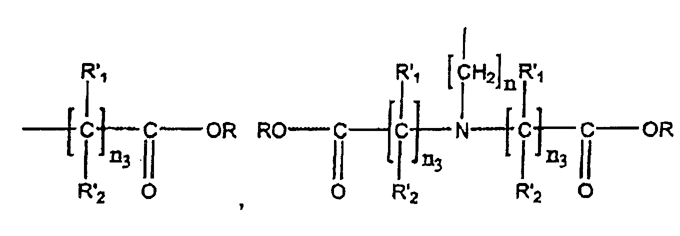 und 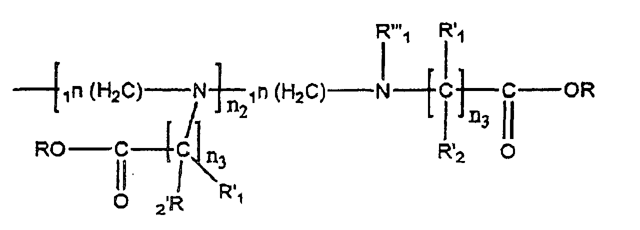
ausgewählt ist, worin jeder Rest R'''₁ unter Wasserstoff, einer Alkylgruppe, einer Arylgruppe ausgewählt ist, und
worin jedes n, n₁ unabhängig eine ganze Zahl darstellt, welche im Bereich von 1 bis 20 liegt, und
worin jedes n₂ eine ganze Zahl bedeutet, welche im Bereich von 1 bis 6 liegt,
worin jedes n₃ unabhängig eine ganze Zahl darstellt, welche im Bereich von 1 bis 3 liegt, und
worin jeder Rest R unabhängig eine organische Kette einer wirksamen Alkoholkomponente ist, welcher wirksame Alkohol unter Aromaalkoholen und Parfumalkoholen ausgewählt ist; mit der Maßgabe, daß mindestens einer der Reste R₁ und/oder R₂ 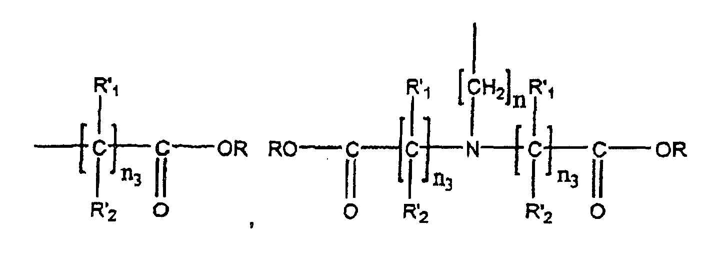
oder 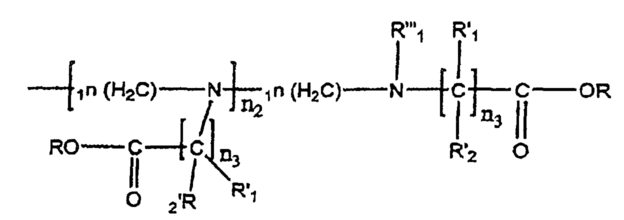 ist, und daß es sich bei der Verbindung nicht um 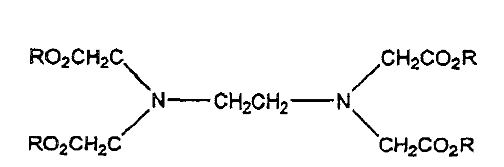 handelt.

2. Verbindung nach Anspruch 1, worin jeder Rest R₁ ein Wasserstoff ist und R₂ 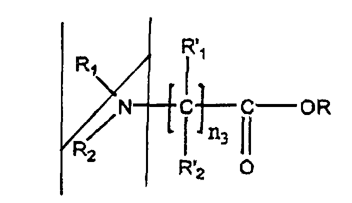 bedeutet.

3. Verbindung nach Anspruch 1, worin jeder Rest R₁, R₂ 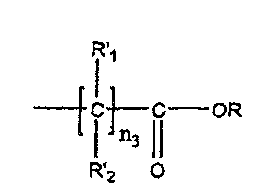 darstellt.

4. Verbindung nach Anspruch 1, worin jeder Rest R₁ 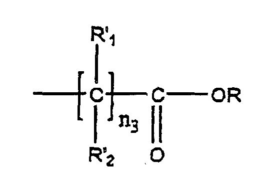 bedeutet und R₂ 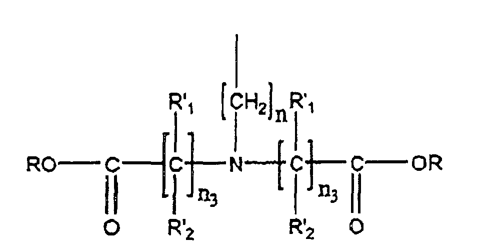 darstellt.

5. Verbindung nach Anspruch 1, worin jeder Rest R₁, R₂ 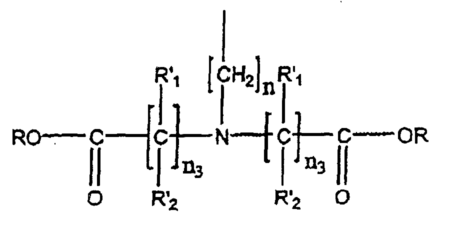 darstellt.

6. Verbindung nach Anspruch 1, worin R₁ 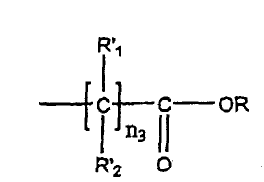 bedeutet und R₂ 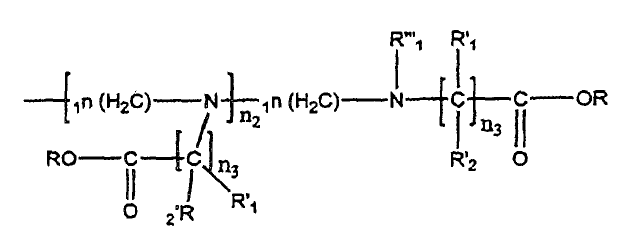 darstellt.

7. Verbindung nach Anspruch 1, worin R₁ and R₂ 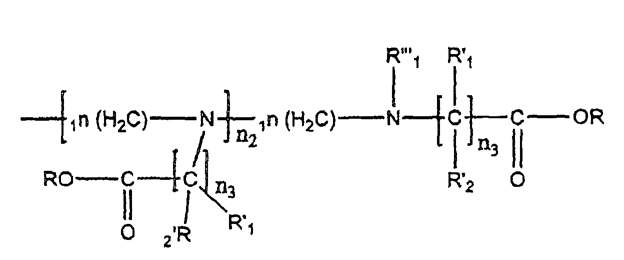 bedeuten.

8. verbindung nach einem der Ansprüche 1 bis 7, worin die genannte R-Gruppe die organische Kette eines Parfumalkohols ist.

9. Verbindung nach Anspruch 8, worin die genannte R-Gruppe die organische Kette eines Parfumalkohols ist, welcher Alkohol unter 2-Phenoxyethanol, Phenylethylalkohol, Geraniol, Citronellol, 3-Methyl-5-phenyl-1-pentanol, 2,4-Dimethyl-3-cyclohexan-1-methanol, Linalool, Tetrahydrolinalool, 1,2-Dihydromyrcenol, Hydroxycitronellal, Farnesol, Menthol, Eugenol, Vanilin, cis-3-Hexanol, Terpineol und Gemischen hievon ausgewählt ist, vorzugsweise Geraniol und/oder Citronellol ist.

10. Verfahren zur Herstellung einer verbindung, wie sie in einem der Ansprüche 1 bis 9 definiert ist, welches die Schritte von:
(a) Verestern des Alkohols zu dessen Halogenacetatesterform in Gegenwart eines Amins, und
(b) Umsetzen des genannten Halogenacetatesters mit dem gewünschten Amin in Gegenwart eines sterisch gehinderten Amins.

11. Verwendung einer Verbindung nach einem der Ansprüche 1 bis 9 als Precursor für die entsprechende Betainesterverbindung.

12. Zusammensetzung zum Wäschewaschen und zur Reinigung, welche eine oder mehrere grenzflächenaktive Komponenten umfaßt und eine Verbindung nach einem der Ansprüche 1 bis 9 einverleibt enthält, wobei die genannte Zusammensetzung alkalisch ist.

13. Zusammensetzung nach Anspruch 12, wobei die genannte Zusammensetzung unter einer Detergenzzusammensetzung, einer Zusammensetzung zur Reinigung von harten Oberflächen und einer Zusammensetzung zum Geschirrspülen ausgewählt ist.

14. Zusammensetzung nach Anspruch 12, wobei die genannte Zusammensetzung eine Zusammensetzung zur Reinigung von harten Oberflächen ist.

15. Verfahren zur Freisetzung einer wirksamen Alkoholkomponente, welches das Inkontaktbringen einer Zusammensetzung, wie sie in Anspruch 12 definiert ist, mit einem Material umfaßt, sodaß mindestens eines der Stickstoffatome des beta-Aminoesters protoniert wird.

16. Verfahren nach Anspruch 15, worin das genannte Material Wasser ist.

17. Verfahren nach einem der Ansprüche 15 oder 16, worin die genannte wirksame Komponente eine Parfumalkoholkomponente ist.

## Revendications

1. Composé de formule: 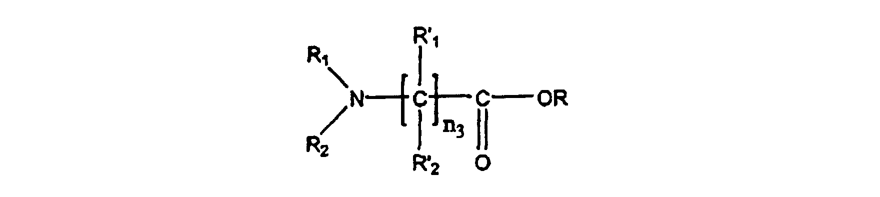 dans laquelle chaque radical R'₁, R'₂ est un atome d'hydrogène et
dans laquelle chaque radical R₁, R₂, indépendamment, est choisi parmi un atome d'hydrogène, un groupe alkyle, un groupe aryle, 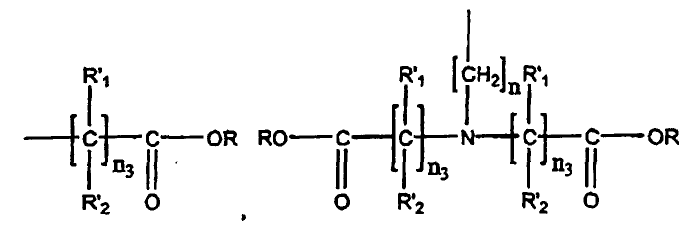 et 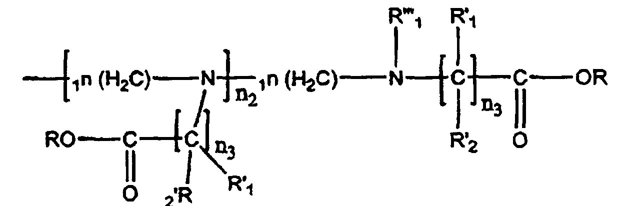
où chaque R'''₁ est choisi parmi un atome d'hydrogène, un groupe alkyle, un groupe aryle, et
où chaque indice n, n₁, indépendamment, est un nombre entier situé dans la gamme de 1 à 20, et
où chaque n₂ est un nombre entier situé dans la gamme de 1 à 6,
où chaque n₃, indépendamment, est un nombre entier situé dans la gamme de 1 à 3, et
où chaque R, indépendamment, est une chaîne organique d'un constituant alcool actif, ledit alcool actif étant choisi parmi les alcools aromatisés et les alcools parfumés; à condition que l'un au moins des radicaux R₁ et/ou R₂ soit: 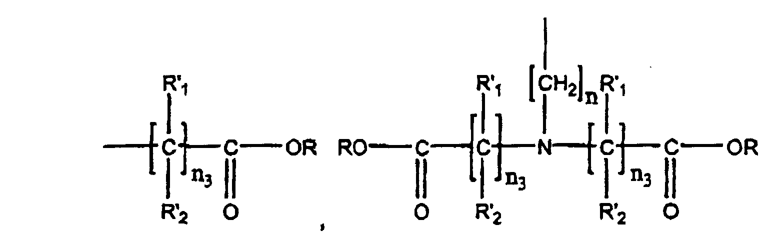
ou 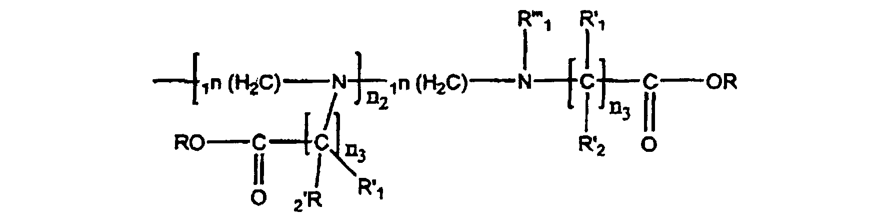 et que le composé ne soit pas:

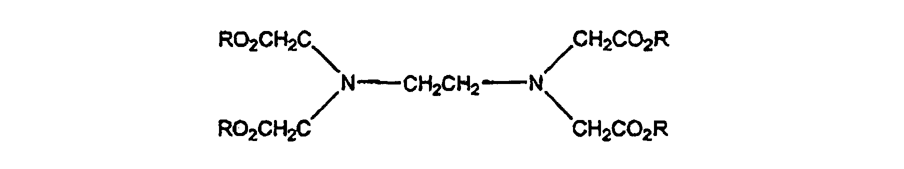

2. Composé selon la revendication 1, dans lequel chaque R₁ est un atome d'hydrogène et R₂ est

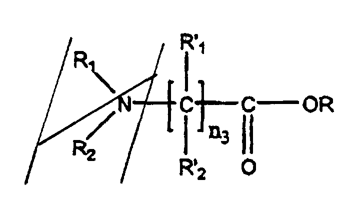

3. Composé selon la revendication 1, dans lequel chaque R₁ et R₂ est

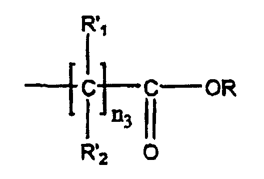

4. Composé selon la revendication 1, dans lequel chaque R₁ est 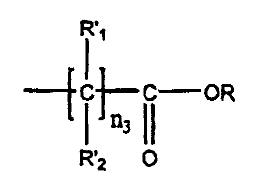 et R₂ est

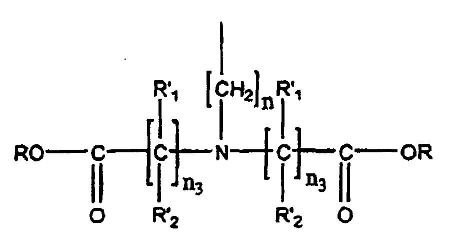

5. Composé selon la revendication 1, dans lequel chaque R₁ et R₂ est

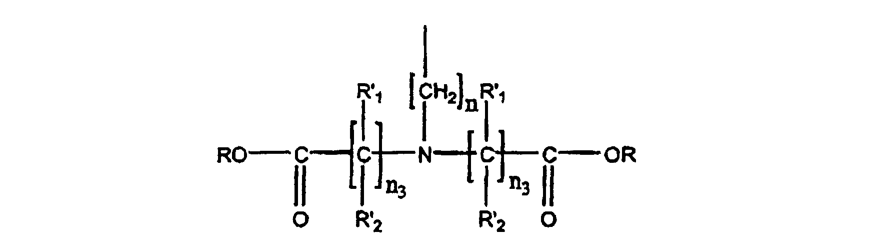

6. Composé selon la revendication 1, dans lequel chaque R₁ est 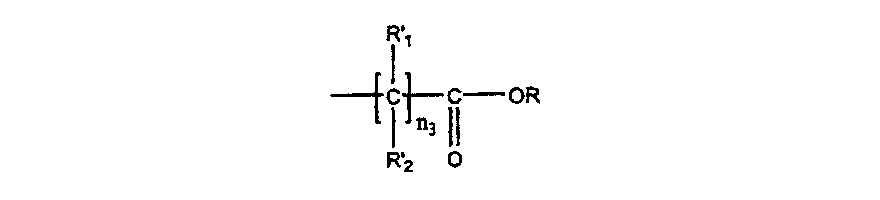 et R₂ est

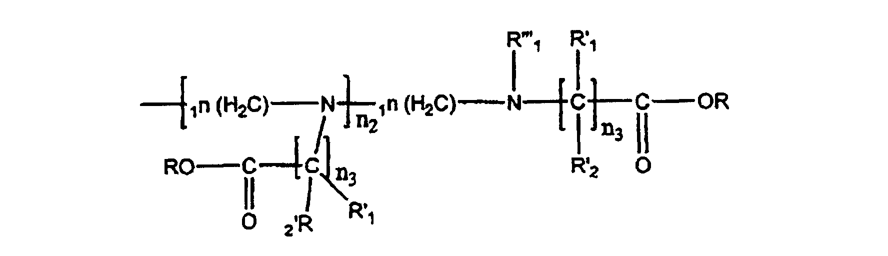

7. Composé selon la revendication 1, dans lequel chaque R₁ et R₂ sont:

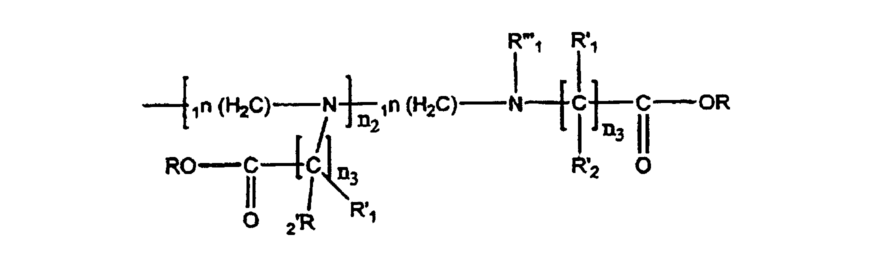

8. Composé selon l'une quelconque des revendications 1-7, dans lequel ledit groupe R est la chaîne organique d'un alcool parfumé.

9. Composé selon la revendication 8 dans lequel ledit groupe R est la chaîne organique d'un alcool parfumé, ledit alcool étant choisi parmi le 2-phénoxyéthanol, l'alcool phényléthylique, le géraniol, le citronellol, le 3-méthyl-5-phényl-1-pentanol, le 2,4-diméthyl-3-cyclohexane-1-méthanol, le linalol, le tétrahydrolinalol, le 1,2-dihydromyrcénol, l'hydroxycitronellal, le farnésol, le menthol, l'eugénol, la vanilline, le cis-3-hexanol, le terpinéol et leurs mélanges, de préférence le géraniol et/ou le citronellol.

10. Procédé de préparation d'un composé tel que défini dans l'une quelconque des revendications 1-9 qui comprend les étapes consistant à:
(a) estérifier l'alcool en sa forme ester halogénoacétate en présence d'une amine, et
(b) faire réagir ledit ester halogénoacétate avec l'amine souhaitée en présence d'une amine à empêchement stérique.

11. Utilisation d'un composé selon l'une quelconque des revendications 1-9 comme précurseur du composé ester de bétaine correspondant.

12. Composition de lessive et de nettoyage comprenant un ou plusieurs constituants tensioactifs et renfermant un composé selon l'une quelconque des revendications 1-9, dans laquelle ladite composition est alcaline.

13. Composition selon la revendication 12 dans laquellc ladite composition est choisie parmi une composition détergente, une composition de nettoyage des surfaces dures et une composition de lavage de la vaisselle.

14. Composition selon la revendication 12 dans laquelle ladite composition est une composition de nettoyage des surfaces dures.

15. Procédé pour libérer un constituant alcool actif qui comprend la mise en contact d'une composition telle que définie dans la revendication 12 avec une matière de telle sorte que l'un au moins des atomes d'azote du β-aminoester soit protoné.

16. Procédé selon la revendication 15 dans lequel ladite matière est l'eau.

17. Procédé selon l'une quelconque des revendications 15 et 16 dans lequel ledit constituant actif est un constituant alcool parfumé.
